(19) Europäisches Patentamt
European Patent Office
Office européen des brevets

(11) Publication number: **0 308 101 B1**

(12) **EUROPEAN PATENT SPECIFICATION**

(45) Date of publication of patent specification: **22.07.92**

(51) Int. Cl.5: **C07C 29/132**, C07C 29/50, C07C 31/12

(21) Application number: **88308061.6**

(22) Date of filing: **31.08.88**

(54) **Preparation of tertiary butyl alcohol.**

(30) Priority: **08.09.87 US 94171**
**08.09.87 US 94172**
**08.09.87 US 94173**
**08.09.87 US 94174**
**08.09.87 US 94170**
**08.09.87 US 94175**
**08.09.87 US 94352**

(43) Date of publication of application:
**22.03.89 Bulletin 89/12**

(45) Publication of the grant of the patent:
**22.07.92 Bulletin 92/30**

(84) Designated Contracting States:
**DE FR GB NL**

(56) References cited:
**EP-A- 0 079 705**
**DE-A- 3 248 465**
**GB-A- 1 213 822**
**US-A- 4 028 423**
**DE-A- 1 128 847**
**GB-A- 1 212 824**
**US-A- 3 816 548**

(73) Proprietor: **TEXACO DEVELOPMENT CORPORATION**
**2000 Westchester Avenue**
**White Plains, New York 10650(US)**

(72) Inventor: **Sanderson, John Ronald**
**P.O. Box 587**
**Leander Texas 78741(US)**
Inventor: **Knifton, John Fredrick**
**10900 Catskill Trail**
**Austin 78750(US)**
Inventor: **Marquis, Edward Thomas**
**9004 Collinfield Drive**
**Austin 78758(US)**
Inventor: **Mueller, Mark Allan**
**13103 Mansfield Circle**
**Austin 78732(US)**

(74) Representative: **Brock, Peter William et al**
**UROUHART-DYKES & LORD 91 Wimpole Street**
**London W1M 8AH(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to the catalytic decomposition of tertiary butyl hydroperoxide. More particularly, this invention relates to a method for the preparation of tertiary butyl alcohol by the catalytic decompositon of tertiary butyl hydroperoxide.

It is known from the prior art cited below to react isobutane with oxygen, either thermally or catalytically, to form a peroxidation reaction product wherein the principal peroxide that is formed is tertiary butyl hydroperoxide. It is also known to thermally or catalytically decompose the tertiary butyl hydroperoxide to form tertiary butyl alcohol.

Taylor et al. U.S. Patent No. 4,551,553 is directed to a process for the formation of alcohols such as tertiary butyl alcohol by the catalytic decomposition of an organic hydroperoxide such as tertiary butyl hydroperoxide using a binary catalyst composed of a mixture of a ruthenium compound with a chromium compound. It is stated that the use of the binary catalyst eliminates the need for stabilizing ligands.

The metal phthalocyanines are known compounds, described for example in the ACS Monograph Series by F. H. Moser and A. L. Thomas entitled "Phthalocyanine Compounds" (Rhinehold Publishing Corp.).

Williams et al. U. S. Patent No. 3,816,548 is directed to a liquid phase oxidation process for oxidizing an isoparaffin hydrocarbon such as isobutane to an alcohol such as tertiary butyl alcohol in the presence of certain metal phthalocyanine catalysts.

Klein in U. S. Patent No. 3,472,876, discloses the use of cobalt diimine chelates to catalyze the reaction of oxygen with an olefin to form an olefin epoxide.

Quin U. S. Patent No. 2,854,487 discloses a process wherein isopropyl benzene hydroperoxides are catalytically decomposed to form carbonals in the presence of hydrogen and a catalyst composed of palladium supported on activated alumina.

Grane U. S. Patent No. 3,474,151 discloses that tertiary butyl alcohol starts to dehydrate at 450 ° C. and to decompose at a "rapid rate" at temperatures above 475 ° F. Grane discovered, however, that residual quantities of hydroperoxide contaminants present in tertiary butyl alcohol could be thermally decomposed by heating the contaminated tertiary butyl alcohol at a temperature of 375 ° to 475 ° F. for about 1 to 10 minutes.

Grane et al. U. S. Patent No. 4,294,999 discloses a process wherein isobutane is oxidized in a pressured reactor in the presence of a solubilized molybdenum catalyst to provide a mixture of tertiary butyl alcohol, tertiary butyl hydroperoxide, methanol, acetone, and other oxygen-containing compounds. The tertiary butyl hydroperoxide is thermally decomposed under pressure at about 280 ° F. to provide a tertiary butyl alcohol product containing only residual quantities of tertiary butyl hydroperoxide which are then decomposed in accordance with Grane U. S. Patent No. 3,474,151 by heating the tertiary butyl alcohol at 375 ° to 475 ° for about 1 to 10 minutes. Heating tertiary butyl alcohol containing small amounts of peroxides at high temperatures for even short periods of time to remove the peroxides produces undesirable products such as isobutylene.

Grane et al. U. S. Patent No. 4,296,262 discloses a related process wherein isobutane is reacted with oxygen in a reaction zone for a residence time of about 1 to 10 hours at a temperature of about 240 ° to about 340 ° F. and a pressure of about 100 to about 1000 psig (690 to 69000 kPa g.p, wherein g.p = guage pressure) in the presence of a catalytically effective amount of a soluble molybdenum catalyst. A liquid stream comprising tertiary butyl alcohol is recovered from the reaction mixture and fed to a decomposition zone wherein the tertiary butyl hydroperoxide contained therein is decomposed by "hot aging" at 250-350 ° F. at a pressure lower than the pressure in the oxidation zone. The tertiary butyl alcohol can be further subjected to a clean-up treatment at 375-475 ° F. for 1 to 10 minutes. Worrell et al. in U. S. Patent No. 4,296,263 disclose a related process wherein the feedstock is a mixture of normal butane with isobutane and wherein the oxidation catalyst is a soluble form of chromium, cobalt, nickel, manganese, molybdenum, or a mixture thereof.

When isobutane is reacted with molecular oxygen, the principal products of the reaction are tertiary butyl alcohol and tertiary butyl hydroperoxide. However, minor amounts of other peroxides, including ditertiary butyl peroxide are also formed. Generally speaking, from 10 to 100 parts of tertiary butyl hydroperoxide are formed per part of ditertiary butyl peroxide. Minor quantities of other contaminants are also formed.

In addition, a minor amount of water will be formed, which will normally amount to 0.5 to 1 wt.% of the reactor effluent. The amount of byproduct water that is produced is a function of the severity of the reaction conditions employed and will tend to increase as the severity of the reaction conditions is increased.

A listing of the components present in a representative reaction product, and their nominal boiling

points is given in Table A.

TABLE A

| Component | NBP (° C) |
|---|---|
| Isobutane | -11.7 |
| Methyl formate | 31.8 |
| Acetone | 56.3 |
| Isobutylene oxide | 60.0 |
| Isobutyraldehyde | 64.1 |
| Methanol | 64.7 |
| Methyl-t-butyl peroxide | 74.2 |
| Isopropyl alcohol | 82.3 |
| Tertiary butyl alcohol | 82.4 |
| Ditertiary butyl peroxide | 111.0 |
| t-butyl-i-pr-peroxide | 124.0 |
| Tertiary butyl formate | 163.8 |

The minor by-products are sometimes difficult to remove. For example, tertiary butyl formate has a higher boiling point than ditertiary butyl hydroperoxide but tends to distill overhead, which suggests that it forms a minimum boiling azeotrope with another component or components.

As indicated, tertiary butyl hydroperoxide is useful as a raw material for the manufacture of tertiary butyl alcohol. The tertiary butyl alcohol can be formed by catalytic decomposition of the tertiary butyl hydroperoxide. In the Williams et al. process disclosed in U. S. Patent No. 3,472,876, an oxygen-containing gas was charged to a reactor containing isobutane and an oxidation catalyst to provide a reaction mixture comprising tertiary butyl alcohol, tertiary butyl hydroperoxide, acetone, and tertiary butyl ether. The reported results in the patent indicate that there was a comparatively low rate of conversion and a comparatively poor selectivity of the reaction to tertiary butyl alcohol.

## SUMMARY OF THE INVENTION

In accordance with the present invention, isobutane is reacted with oxygen in an oxidation zone to provide an oxidation product comprising a solution of tertiary butyl hydroperoxide in unreacted isobutane. A catalyst may be present to catalyze the reaction of the oxygen with the isobutane if desired.

A suitable feedstock is used, such as one prepared by the oxidation of isobutane with molecular oxygen to provide an oxidation reaction product containing a solution of tertiary butyl hydroperoxide in unreacted isobutane. The feedstock may comprise tertiary butyl hydroperoxide dissolved in tertiary butyl alcohol which is recovered from the oxidation reaction product. The feedstock is charged to a catalytic decomposition zone wherein the tertiary butyl hydroperoxide is decomposed in the presence of a soluble ruthenium catalyst compound promoted with a bidentate ligand to provide a decomposition reaction product characterized by a high conversion rate and a high selectivity of tertiary butyl hydroperoxide to tertiary butyl alcohol.

The tertiary butyl alcohol will not be the only decomposition product that is formed. A minor amount of ditertiary butyl peroxide will also be formed together with other oxygen-containing materials such as those listed above.

The tertiary butyl alcohol that is recovered from the decomposition reaction mixture will be contaminated with ditertiary butyl peroxide and other oxygenated impurities.

The ditertiary butyl peroxide can be recovered, if desired, by a process such as the process disclosed in copending Sanderson et al. U. S. patent application S.N. 06/945,628, filed December 23, 1986, and entitled "Recovery of Purified Ditertiary Butyl Peroxide" or the process disclosed in copending application S.N. 06/945,629, filed December 23, 1986 by Sanderson et al., and entitled "Ditertiary Butyl Peroxide Recovery".

If desired, the ditertiary butyl peroxide and other contaminants may be removed from the tertiary butyl alcohol product by a catalytic purification process such as, for example, the purification process disclosed in copending Sanderson et al. U. S. patent application S.N. 06/836,798, filed March 6, 1986, and entitled "Removal of Peroxide Contaminants from Tertiary Butyl Alcohol Using a Nickel Catalyst", or by a purification process disclosed in copending Sanderson et al. U. S. patent application S.N. 06/926,159, filed November 3, 1986, and entitled "Catalytic Removal of Peroxide Contaminants from Tertiary Butyl Alcohol"

entitled "Catalytic Decomposition of Impurities in Tertiary Butyl Alcohol" or, as yet another example, by the process disclosed in copending Sanderson et al. U.S. patent application S.N. 07/004,508, filed January 30, 1987, and entitled "Catalyst for Removing Peroxide Contaminants from Tertiary Butyl Alcohol".

According to the present invention, there is provided a method for converting t-butyl hydroperoxide to decomposition products, principally t-butyl alcohol, by bringing a t-butyl hydroperoxide charge stock into contact with a catalytically effective amount of a catalyst in a reaction zone in liquid phase with agitation wherein the catalyst is selected from:

(a) a metal porphine compound
(b) a soluble ruthenium catalyst compound promoted with a bidentate ligand and
(c) a promoted metal phthalocyanine compound.

The metal porphine compound can be an iron (III) or manganese (III) porphine compound. Conversion and/or selectivity are enhanced by using an appropriate mercaptan and/or an amine.

A solvent which may be used in conjunction with the metal porphine may be any suitable organic solvent in which tertiary butyl hydroperoxide is soluble at least to an extent sufficient to provide a solution containing from 5 to 50 wt.% of tertiary butyl hydroperoxide. A preferred solvent is isobutane and a still more preferred solvent is tertiary butyl alcohol or a mixture thereof with isobutane. In accordance with a particularly preferred embodiment of a process using metal porphine the charge material for the process will comprise about a 5 to about a 30 wt.% solution of tertiary butyl hydroperoxide and teriary butyl alcohol.

The metal porphine catalysts used are suitably a porphine of a heavy metal such as, for example, iron (III) meso-tetraphenyl porphine chloride [Fe(III)TPPCl] or manganese (III) meso-tetraphenyl porphine acetate [Mn(III)TPPOAc].

The porphine ring consists essentially of four pyrrole rings

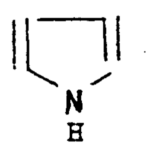

united by methylene groups. When a phenyl group replaces a hydrogen in the methylene group, tetraphenyl porphine (TPP) is the compound.

Tetraphenyl porphine (TPP) may be formed by reaction of benzaldehyde with pyrrole by methods known in the art. The two hydrogens in the center may be replaced by metals such as iron or manganese ions.

Aldrich Catalog (1986-1987), p. 1253, gives a partial listing of the available metal porphines. The metal tetraphenyl porphines are preferred over unsubstituted porphines since they are more readily available and more stable, including compounds such as 5,10,15,20-tetraphenyl-21H,23H-porphine; 5,l0,l5,20-tetraphenyl-21H,23H-porphine copper; 5,10,15,20-tetraphenyl-21H,23H-porphine iron (III) chloride; 5,10,15,20-tetraphenyl-21H,23H-porphine magnesium; 5,10,15,20-tetraphenyl-21H,23H-porphine manganese (III) chloride; 5,10,15,20-tetraphenyl-21H,23H-porphine nickel (II); 5,10,15,20-tetraphenyl-21H,23H-porphine palladium (II); 5,10,15,20-tetraphenyl-21H,23H-porphine ruthenium (II) carbonyl; 5,10,15,20-tetraphenyl-21H,23H-porphine vanadium (IV) oxide; 5,10,15,20-tetraphenyl-21H,23H-porphine zinc.

The yield and selectivity are improved if the catalyst composition also contains an appropriate mercaptan and/or an appropriate amine.

By way of example, mercaptans that may be used include compounds such as $C_1$ to $C_{18}$ alkyl thiols such as dodecane thiol, butane thiol, hexane thiol, decane thiol, octadecane thiol.

Examples of appropriate amines that may be used include compounds such as pyridine, isoquinoline, imidazole, 1-alkyl or 2-alkyl imidazoles wherein the alkyl group contains 1 to 4 carbon atoms, such as 1-methyl imidazole, 2-methyl imidazole, quinoline.

A catalytically effective amount of the metal porphine (e.g., iron (III) and/or manganese (III) porphine) should be used such as, for example, from 0.001 to 5 wt.%, based on the weight of the tertiary butyl hydroperoxide charge and, more preferably, 0.01 to 2 wt.%. In accordance with customary practice, from 0.2 to 5 parts by weight of mercaptan per part of porphine catalyst may be used, as may from 0.2 to 5 parts by weight of amine per part of porphine catalyst.

**USE OF A METAL PORPHINE**

The process may be conducted batchwise in kettles or by continuously passing the reactants through a reactor.

The catalytic decomposition of the tertiary butyl hydroperoxide is preferably conducted at a temperature within the range of 20° to 125°C. and, more preferably, at a temperature within the range of 30 to 60°C. The reaction is preferably conducted at autogeneous pressure although superatmospheric pressures up to about 1000 psig. (6900 kPa g.p.) may be used, if desired.

Flow rates of the charge solution to the reaction zone should be adjusted in order to provide an appropriate contact time within the reactor. In a batch process, the holding time may suitably be from 0.5 to 10 hours.

In accordance with the more preferred embodiment isobutane is reacted with oxygen in an oxidation zone under oxidation reaction conditions including a temperature of 135° to 155°C., a pressure of 300 to 800 psig. (5520 kPa g.p.), and a holding time of 0.5 to 5 hours to provide an initial oxidation reaction product comprising unreacted isobutane, tertiary butyl hydroperoxide, and some tertiary butyl alcohol. The oxidation reaction product is fractionated in any appropriate manner (e.g., by distillation in a distillation zone) to remove the isobutane therefrom for recycle and to provide a solution of tertiary butyl hydroperoxide and tertiary butyl alcohol which will normally contain from 10 to 40 wt.% of tertiary butyl hydroperoxide. If the tertiary butyl hydroperoxide concentration is excessive, additional tertiary butyl alcohol may be added.

The solvent solution of tertiary butyl alcohol in organic solvents (e.g., tertiary butyl alcohol solvent solution of tertiary butyl hydroperoxide) is then charged to a catalytic hydroperoxide decomposition zone where it is brought into contact with an iron III and/or manganese III porphine catalyst to substantially selectively convert the tertiary butyl hydroperoxide to tertiary butyl alcohol with high yields and selectivities.

The reaction product from the tertiary butyl hydroperoxide decomposition step may then be fractionated in any suitable manner, such as, for example, in the manner shown in the process disclosed in above identified copending U. S. patent application S.N. 06/945,628 filed December 23, 1986. In accordance with a process recovery sequence of this nature, both the tertiary butyl alcohol and the ditertiary butyl peroxide will be recovered in purified form as products of the reaction.

Alternately, a crude tertiary butyl alcohol product stream contaminanted with ditertiary butyl peroxide and other contaminants may be obtained which will then be further treated either thermally, in accordance with the process of the Grane et al. U. S. patents, or catalytically by one of the processes disclosed in the copending Sanderson et al. patent applications to convert the ditertiary butyl peroxide to tertiary butyl alcohol and to otherwise significantly reduce the level of contamination of the other oxygen-containing impurities.

SPECIFIC EXAMPLES (METAL PORPHINE)

The invention will be further illustrated by the following specific examples which are given by way of illustration and not as a limitation of this invention.

Procedure-1: Tube Experiments

A 150-ml Fisher-Porter pressure tube equipped with pressure gauge, rupture disk, and shut-off valve was charged with 15.0g of a 20% TBHP solution in TBA, and catalyst(s). The tube was suspended in a constant temperature bath (+ or - 0.2°C.) for the desired period of time at the required temperature. The tube was shaken from time to time during the run. At the end of the run, the tube was placed in cold water (15-20°C.) for 15 minutes. The pressure was then slowly released. The contents were analyzed by GC. The results are shown in the attached Table I.

Procedure-2

A 200 ml round-bottomed flask equipped with magnetic stirrer, water bath, water cooled condenser and thermometer was charged with 20.0g 20% solution TBHP in TBA. The reaction mixture was vigorously stirred and catalyst(s) added all at once. 1.0 (one) µl aliquots were withdrawn at designated times and anlyzed at once by GC. The results are shown in the attached table.

TABLE I

Decomposition of 20% TBHP (in TBA) in the Presence of Various Catalysts

| N.B. Number | Catalyst(s)[a] | Time (Hr) | Temp (°C) | TBHP | Wt.% TBA | Wt.% Acetone | Wt.% MeOH | Wt.% DTBP |
|---|---|---|---|---|---|---|---|---|
| 6224-77 | $Ru(AcAc)_3$ [.005g] | 1.0 | 60.0 | 0.024 | 96.13 | 1.48 | 0.12 | 1.24 |
| 6225-08 | Mn(III)TPPOAc[.01g] | 2.0 | 60.0 | 0.048 | 96.36 | 1.38 | 0.08 | 1.30 |
| 6225-10 | Fe(III)TPPCl[.01g] | 2.0 | 60.0 | 0.012 | 96.12 | 1.73 | 0.14 | 0.96 |
| 6225-34 | Mn(III)TPPOAc[.01g] | 1.0 | 60.0 | 0.039 | 96.35 | 1.39 | 0.089 | 1.29 |
| 6225-38 | Mn(III)TPPOAc[.01g] | 2.0 | 40.0 | 2.56 | 93.89 | 0.53 | 0.023 | 2.32 |
| 6225-39 | Mn(III)TPPOAc[.01g] Py [.05g] | 2.0 | 40.0 | 0.057 | 95.76 | 0.89 | 0.03 | 2.31 |
| 6225-40 | Mn(III)TPPOAc[.01g] Py [.05g] | 2.0 | 40.0 | ~0 | 96.03 | 0.88 | <0.1 | 2.29 |
| 6225-42 | Mn(III)TPPOAc[.01g] QN [.02g] | 1.0 | 40.0 | 0.073 | 96.29 | 0.83 | <0.1 | 2.26 |
| 6225-44 | Mn(III)TPPOAc[.01g] IQN [.01g] | 2.0 | 40.0 | ~0 | 96.33 | 0.86 | <0.1 | 2.27 |
| 6225-47 | Fe(III)TPPCl[.01g] Py [.017g] | 2.0 | 40.0 | 0.41 | 95.71 | 0.77 | <0.1 | 2.58 |
| 6225-27[b] | $Ru(AcAc)_3$ [.005g] $Cr(AcAc)_3$ [.01g] | 2.0 | 60.0 | 0.051 | 96.37 | 1.41 | 0.097 | 1.13 |
| — | Starting Material | | | 20.20 | 79.28 | 0.007 | 0 | 0.06 |

(a) AcAc = Acetylacetonate; TPP = Tetraphenylporphine; OAc = Acetate; Py = Pyridine; QN = Quinoline; IQN = Isoquinoline

(b) U. S. Patent 4,551,553 (ARCO)

With reference to Table I, it will be noticed that the use of amines resulted in an enhanced rate of decomposition of the tertiary butyl hydroperoxide as shown by the increased conversion for the given reaction time and an enhanced selectivity of the tertiary butyl hydroperoxide to tertiary butyl alcohol.

At high temperatures (60 °C), it will be noted that <0.04% TBHP remains after 1-2 hours reaction with both Fe and Mn tetraphenyl porphine. Note that the low value by-product acetone >1% under these conditions [see exp. 6225-08, -10, -34]. $Ru(AcAc)_3$ [6224-77] is shown for comparison. At lower temperatures (40 °C) [6225-38], there is substantial TBHP remaining after 2 hours. In the presence of pyridine under the same conditions, there is only a small amount of TBHP remaining [6225-39, -40]. Furthermore, the low value by-product acetone has been reduced to <1%. Quinoline and isoquinoline show the same effect when used in combination with the metal phthalocyanines of this invention. [See 6225-42, -44.] For comparison, an example was run using the catalyst of U. S. Patent No. 4,551,553 [see 6225-27]. Note that the low value

by-product acetone >1% with the catalyst of this system.

TABLE II

Decomposition of TBHP in the Presence of Various Catalysts

| Notebook Number | Time (Hrs) | Temp (°C) | Catalysts[a] | Reactor[b] | TBHP | Products, Wt.% TBA | Acetone | Methanol | DTBP |
|---|---|---|---|---|---|---|---|---|---|
| 6225-59 | .25 | 25.0 | MN(III)TPPOAc (.01g) IM (.01g) | F | 15.37 | 83.21 | 0.095 | <0.1 | 0.91 |
| 0059-02 | .75 | 25.0 | MN(III)TPPOAc (.01g) IM (.01g) | F | 9.16 | 88.24 | 0.21 | <0.1 | 1.95 |
| 0059-03 | 1.25 | 25.0 | MN(III)TPPOAc (.01g) IM (.01g) | F | 7.88 | 89.46 | 0.24 | <0.1 | 2.13 |
| 0059-04 | 1.50 | 25.0 | MN(III)TPPOAc (.01g) IM (.01g) | F | 7.36 | 89.80 | 0.22 | <0.1 | 2.18 |
| 0059-05 | 2.50 | 25.0 | MN(III)TPPOAc (.01g) IM (.01g) | F | 6.77 | 90.26 | 0.27 | <0.1 | 2.26 |
| 0059-06 | 3.75 | 25.0 | MN(III)TPPOAc (.01g) IM (.01g) | F | 6.43 | 90.47 | 0.29 | <0.1 | 2.34 |
| 6225-61 | .15 | 25.0 | MN(III)TPPOAc (.02g) IM (.02g) | F | 8.18 | 89.12 | 0.24 | <0.1 | 1.99 |
| 0061-02 | .57 | 25.0 | MN(III)TPPOAc (.02g) IM (.02g) | F | 1.73 | 94.41 | 0.45 | <0.1 | 2.72 |
| 0061-03 | 1.03 | 25.0 | MN(III)TPPOAc (.02g) IM (.02g) | F | 1.12 | 94.89 | 0.51 | <0.1 | 2.85 |
| 0061-04 | 1.58 | 25.0 | MN(III)TPPOAc (.02g) IM (.02g) | F | 1.28 | 94.91 | 0.53 | <0.1 | 2.84 |
| 0061-05 | 2.00 | 25.0 | MN(III)TPPOAc (.02g) IM (.02g) | F | 1.09 | 95.02 | 0.56 | <0.1 | 2.88 |
| 0061-06 | 2.60 | 25.0 | MN(III)TPPOAc (.02g) IM (.02g) | F | 0.91 | 95.15 | 0.58 | <0.1 | 2.91 |
| 0061-07 | 4.67 | 25.0 | MN(III)TPPOAc (.02g) IM (.02g) | F | 0.62 | 95.42 | 0.59 | <0.1 | 2.90 |
| 6225-62 | .08 | 25.0 | MN(III)TPPOAc (.04g) IM (.04g) | F | 2.61 | 94.09 | 0.50 | <0.1 | 2.36 |
| 0062-02 | .50 | 25.0 | MN(III)TPPOAc (.04g) IM (.04g) | F | 0.27 | 95.91 | 0.70 | <0.1 | 2.60 |
| 0062-03 | 1.00 | 25.0 | MN(III)TPPOAc (.04g) IM (.04g) | F | 0.06 | 96.07 | 0.74 | <0.1 | 2.61 |
| 6225-63 | .10 | 10.0 | MN(III)TPPOAC (.04g) IM (.04g) | F | 15.49 | 83.07 | 0.08 | <0.1 | 0.91 |
| 0063-02 | .50 | 10.0 | MN(III)TPPOAc (.04g) IM (.04g) | F | 14.94 | 83.11 | 0.10 | <0.1 | 1.45 |
| 0063-03 | .92 | 10.0 | MN(III)TPPOAc (.04g) IM (.04g) | F | 12.24 | 85.24 | 0.13 | <0.1 | 2.13 |
| 0063-04[c] | 16.0 | 25.0 | MN(III)TPPOAc (.04g) IM (.04g) | F | 0.02 | 94.36 | 0.43 | <0.1 | 4.37 |
| 6225-65 | .15 | 33.0 | MN(III)TPPOAc (.04g) IM (.04g) | F | 4.34 | 92.37 | 0.35 | <0.1 | 2.60 |
| 0065-02 | .55 | 29.0 | MN(III)TPPOAc (.04g) IM (.04g) | F | 1.07 | 94.98 | 0.50 | <0.1 | 3.03 |
| 0065-03 | 1.06 | 27.0 | MN(III)TPPOAc (.04g) IM (.04g) | F | 0.36 | 95.46 | 0.57 | <0.1 | 3.15 |
| 0065-04 | 2.00 | 28.0 | MN(III)TPPOAc (.04g) IM (.04g) | F | 0.12 | 95.73 | 0.59 | <0.1 | 3.11 |

TABLE II (Cont'd.)

| Notebook Number | Time (Hrs) | Temp (°C) | Catalysts[a] | Reactor[b] | Products, Wt.% TBHP | TBA | Acetone | Methanol | DTBP |
|---|---|---|---|---|---|---|---|---|---|
| 6225-69 | .12 | 37.0 | MN(III)TPPOAc (.04g) IM (.08g) | F | 1.32 | 95.02 | 0.46 | <0.1 | 2.78 |
| 0069-02 | .50 | 26.0 | MN(III)TPPOAc (.04g) IM (.08g) | F | 0.13 | 95.94 | 0.61 | <0.1 | 2.88 |
| 6225-83 | .20 | 25.4 | MN(III)TPPOAc(.04g)IM(.04g)DT(.17g) | F | 14.48 | 84.04 | 0.089 | <0.1 | 0.96 |
| 0083-02 | .67 | 24.3 | MN(III)TPPOAc(.04g)IM(.04g)DT(.17g) | F | 12.32 | 85.78 | 0.12 | <0.1 | 1.36 |
| 0083-03 | 1.60 | 25.0 | MN(III)TPPOAc(.04g)IM(.04g)DT(.17g) | F | 11.15 | 86.72 | 0.15 | <0.1 | 1.57 |
| 0083-04 | 3.50 | 24.5 | MN(III)TPPOAc(.04g)IM(.04g)DT(.17g) | F | 10.58 | 87.28 | 0.17 | <0.1 | 1.70 |
| 0083-05 | 21.5 | 25.0 | MN(III)TPPOAc(.04g)IM(.04g)DT(.17g) | F | 9.34 | 88.22 | 0.21 | <0.1 | 1.93 |
| 6225-85 | .13 | 30.5 | MN(III)TPPOAc(.04g)IM(.08g)DT(.08g) | F | 5.15 | 91.95 | 0.26 | <0.1 | 2.32 |
| 0085-02 | .58 | 25.0 | MN(III)TPPOAc(.04g)IM(.08g)DT(.08g) | F | 1.22 | 95.07 | 0.43 | <0.1 | 2.89 |
| 0085-03 | 1.06 | 25.0 | MN(III)TPPOAc(.04g)IM(.08g)DT(.08g) | F | 0.60 | 95.56 | 0.40 | <0.1 | 3.04 |
| 0085-04 | 3.33 | 25.0 | MN(III)TPPOAc(.04g)IM(.08g)DT(.08g) | F | 0.13 | 95.80 | 0.54 | 0.03 | 3.02 |
| 6225-88 | .17 | 35.5 | MN(III)TPPOAc(.04g)IM(.08g)DT(.17g) | F | 4.75 | 92.40 | 0.31 | 0.06 | 1.97 |
| 0088-02 | .62 | 27.0 | MN(III)TPPOAc(.04g)IM(.08g)DT(.17g) | F | 2.20 | 94.53 | 0.42 | 0.05 | 2.39 |
| 0088-03 | 2.00 | 25.8 | MN(III)TPPOAc(.04g)IM(.08g)DT(.08g) | F | 1.16 | 95.40 | 0.42 | 0.05 | 2.55 |
| 6225-93 | .18 | 36.6 | MN(III)TPPOAC(.04g)IM(.12g) | F | 0.77 | 95.71 | 0.62 | <0.1 | 2.49 |
| 0093-02 | .62 | 28.0 | MN(III)TPPOAc(.04g)IM(.12g) | F | 0.10 | 96.35 | 0.61 | <0.1 | 2.54 |
| -- | | | Starting Material | | 20.20 | 79.28 | 0.007 | 0 | 0.06 |

(a) TPP = meso-tetraphenylporphine; OAC = acetate; IM = imidazole; AcAc = acetylacetonate; DT = dodecane thiol

(b) T = 150 ml Fisher-Porter tube, 15.0g scale
F = 200 ml round-bottomed flask, 20.0g scale

(c) Allowed to warm to 25°C. overnight

Table II shows some experiments with imidazole and manganese (III) tetraphenyl porphine acetate. Note here also that the acetone remains below 1%. Exp. No. 0061-07 shows after 4.67 hours at 25 ° C, 0.62 wt.% TBHP remaining and only 0.59% of the low value by-product acetone. Higher concentrations of catalyst produce even lower concentrations of TBHP and the present acetone remains significantly below 1%. See 0062-03, 0063-04, 0065-04 and 0069-02.

## USE OF A RUTHENIUM CATALYST

Alternatively, a tertiary butyl hydroperoxide feedstock is reacted in the presence of the soluble ruthenium catalyst, a bidentate ligand and a solvent.

The ruthenium-containing compounds employed as a catalyst may take many different forms. For instance, the ruthenium may be added to the reaction mixture in an oxide form, as in the case of, for example, ruthenium (IV) oxide hydrate, anhydrous ruthenium (IV) dioxide and ruthenium (VIII) tetraoxide. Alternatively, it may be added as the salt of a mineral acid, as in the case of ruthenium (III) chloride hydrate, ruthenium (III) bromide, ruthenium (III) iodide, tricarbonylruthenium nitrate, or as the salt of a suitable organic carboxylic acid, for example, ruthenium (III) acetate, ruthenium naphthenate, ruthenium valerate and ruthenium complexes with carbonyl-containing ligands such as ruthenium (III) acetylacetonate. The ruthenium may also be added to the reaction zone as a carbonyl or hydrocarbonyl derivative. Here, suitable examples include, among others, triruthenium dodecacarbonyl and other hydrocarbonyls such as $H_2Ru_2(CO)_{12}$, and substituted carbonyl species such as the tricarbonylruthenium (II) chloride dimer, $(Ru(CO)_3Cl_2)_2$.

Ruthenium-containing compounds include oxides of ruthenium, ruthenium salts of an organic carbonylic acid and ruthenium carbonyl or hydrocarbonyl derivatives. Among these are ruthenium (IV) dioxide hydrate, ruthenium (VIII) tetraoxide, anhydrous ruthenium (IV) oxide, ruthenium acetate, ruthenium propionate ruthenium (III) acetylacetonate, and triruthenium dodecacarbonyl.

Additional examples of ruthenium compounds include ruthenium octoate, ruthenium laurate, ruthenium stearate, ruthenium linoleate, ruthenium nitrate, ruthenium sulfate and ruthenium carbonyl.

Other representative examples of soluble ruthenium catalysts that may be used alone or in admixture include ruthenium salts of carboxylic acids produced in the course of oxidation of the precursor hydrocarbon from which the organic hydroperoxide may have been obtained. Representative examples of organic acids which may be produced in the course of oxidation of the hydrocarbon starting material precursor to the organic hydroperoxide include: acetic, formic and isobutyric acids.

Bidentate ligands of the following general structure may be used:

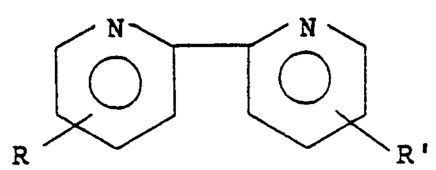

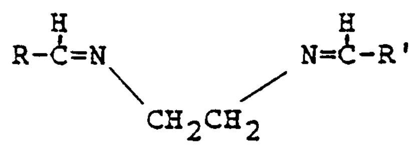

wherein R and R′ may be the same or different and represent hydrogen, methyl, ethyl, propyl, phenyl or chlorophenyl.

Representative examples of amine ligands include amines such as 2,2′-dipyridyl, bis(salicylidene) ethylenediamine, 2,2′-dipicoline, bis(methylsalicylidene) ethylenediamine, bis(ethylsalicylidene) ethylenediamine, bis(propylsalicylidene) ethylenediamine.

From 0.01 to 10 parts by weight of bidentate ligand should be employed per part of ruthenium catalyst and more preferably, from 0.1 to 2 parts.

The concentration of the catalyst in the liquid phase may vary widely. In general, the catalyst system of the present invention may advantageously be employed in amounts ranging from 0.01 ppm to 5,000 ppm of ruthenium.

The solvent to be used in practicing the process of the present invention may be any suitable organic solvent in which tertiary butyl hydroperoxide is soluble at least to an extent sufficient to provide a solution containing from 5 to 30 wt.% of tertiary butyl hydroperoxide. A preferred solvent is isobutane and a still more preferred solvent is tertiary butyl alcohol or a mixture thereof with isobutane. In accordance with the most preferred process involving a soluble ruthenium catalyst, the charge material for the process will comprise a 5 to a 30 wt.% solution of tertiary butyl hydroperoxide and tertiary butyl alcohol.

**EXAMPLES**

The process of the present invention may be conducted batchwise in kettles or by continuously passing the reactants through a reactor.

The catalytic decomposition of the tertiary butyl hydroperoxide is preferably conducted at a temperature within the range of 25° to 125°C. and, more preferably, at a temperature within the range of 30 to 80°C. The reaction is preferably conducted at autogeneous pressure although superatmospheric pressures up to about 1000 psig (6900 kPa g.p.) may be used, if desired.

Flow rates of the charge solution to the reaction zone should be adjusted in order to provide an appropriate contact time within the reactor. In a batch process, the holding time may suitably be from 0.5 to 10 hours.

In accordance with the more preferred embodiment isobutane is reacted with oxygen in an oxidation zone under oxidation reaction conditions including a temperature of 135 to 155°C., a pressure of 300 to 800 psig. (2070 to 5520 kPa g.p.) and a holding time of 2 to 6 hours to provide an initial oxidation reaction product comprising unreacted isobutane, tertiary butyl hydroperoxide, and some tertiary butyl alcohol. The oxidation reaction product is fractionated in any appropriate manner (e.g., by distillation in a distillation zone) to remove the isobutane therefrom for recycle and to provide a solution of tertiary butyl hydroperoxide and tertiary butyl alcohol which will normally contain from about 5 to about 30 wt.% of tertiary butyl hydroperoxide. If the tertiary butyl hydroperoxide concentration is excessive, additional tertiary butyl alcohol may be added.

The solvent solution of tertiary butyl alcohol in organic solvents (e.g., tertiary butyl alcohol solvent solution of tertiary butyl hydroperoxide) is then charged to a catalytic hydroperoxide decomposition zone where it is brought into contact with a catalyst composed of a soluble ruthenium compound and a bidentate ligand to substantially selectively convert the tertiary butyl hydroperoxide to tertiary butyl alcohol with high yields and selectivities.

As indicated, the catalytic decomposition of the tertiary butyl hydroperoxide in the catalytic hydroperoxide decomposition reaction zone may suitably be conducted at a temperature within the range of 25° to 125°C. (and more preferably from 30° to 80°C.) at autogeneous pressure or if desired at a superatmospheric pressure up to 1000 psig. (6900 kPa g.p.) for a contact time within the range of 0.5 to 10 hours.

The reaction product from the tertiary butyl hydroperoxide decomposition step may then be fractionated in any suitable manner, such as, for example, in the manner shown in the process disclosed in above identified copending U. S. patent applicaticn S.N. 06/945,628 filed December 23, 1986. In accordance with a process recovery sequence of this nature, both the tertiary butyl alcohol and the ditertiary butyl peroxide will be recovered in purified form as products of the reaction.

Alternately, a crude tertiary butyl alcohol product stream contaminanted with ditertiary butyl peroxide and other contaminants may be obtained which will then be further treated either thermally, in accordance with the process of the Grane et al. U. S. patents, or catalytically by one of the processes disclosed in the copending Sanderson et al. patent applications to convert the ditertiary butyl peroxide to tertiary butyl alcohol and to otherwise significantly reduce the level of contamination of the other oxygencontaining impurities.

SPECIFIC EXAMPLES (RUTHENIUM CATALYST)

The invention will be further illustrated by the following specific examples which are given by way of illustration and not as limitations on the scope of this invention.

Procedure: Tube Experiments

A 150-ml Fisher-Porter pressure tube equipped with pressure gauge, rupture disk, and shut-off valve was charged with 15.0g of a 20% TBHP solution in TBA, and catalyst(s). The tube was suspended in a constant temperature bath (+ or - 0.2°C.) for the desired period of time at the required temperature. The tube was shaken from time to time during the run. At the end of the run, the tube was placed in cold water (15-20°C.) for 15 minutes. The pressure was then slowly released. The contents were analyzed by GC. The results are shown in the attached Table I.

TABLE I

Decomposition of tert-Butylhydroperoxide in the Presence of a Homogeneous Catalyst System Containing Ruthenium and a Bidentate Ligand

| Notebook No. | Catalyst(s)[a] | Time (Hr) | Temp (°C) | TBHP% | Wt.% TBA | Wt.% Acetone | Wt.% MeOH | Wt.% DTBP |
|---|---|---|---|---|---|---|---|---|
| 6224-63 | $Ru(AcAc)_3$ [.005g] | 1.0 | 100.0 | 0.027 | 95.447 | 2.570 | 0.405 | 0.601 |
| 6224-70 | $Ru(AcAc)_3$ [.005g] | 1.5 | 80.0 | 0.031 | 95.903 | 1.917 | 0.218 | 0.860 |
| 6224-73 | $Ru(AcAc)_3$ [.005g] | 2.0 | 80.0 | 0.031 | 95.956 | 1.883 | 0.209 | 0.890 |
| 6224-75 | $Ru(AcAc)_3$ [.005g] | 4.0 | 60.0 | 0.017 | 96.183 | 1.427 | 0.113 | 1.317 |
| 6224-77 | $Ru(AcAc)_3$ [.005g] | 1.0 | 60.0 | 0.024 | 96.133 | 1.479 | 0.118 | 1.244 |
| 6225-27[b] | $Ru(AcAc)_3$ [.005g] $Cr(AcAc)_3$ [.01g] | 2.0 | 60.0 | 0.051 | 96.370 | 1.410 | 0.097 | 1.135 |
| 6224-100 | $Ru(AcAc)_3$ [.005g] DIPY [.03g] | 2.0 | 60.0 | 1.373 | 96.272 | 1.058 | 0.062 | 0.416 |
| 6225-02 | $Ru(AcAc)_3$ [.005g] DIPY [.01g] | 2.0 | 60.0 | 0.599 | 96.165 | 1.075 | 0.065 | 1.244 |
| 6225-03 | $Ru(AcAc)_3$ [.005g] BSEDA [.01g] | 2.0 | 60.0 | 0.128 | 96.423 | 1.190 | 0.069 | 1.327 |
| 6225-29 | $Ru(AcAc)_3$ [.01g] BSEDA [.02g] | 2.0 | 60.0 | 5.242 | 92.244 | 0.601 | 0.043 | 1.153 |
| 6225-30 | $Ru(AcAc)_3$ [.01g] BSEDA [.01g] | 2.0 | 60.0 | 0.520 | 96.153 | 1.101 | 0.072 | 1.316 |
| 6225-36 | $Ru(AcAc)_3$ [.005g] DIPY [.01g] | 2.0 | 40.0 | 2.349 | 94.159 | 0.570 | 0.021 | 2.144 |
| --- | Starting Material | | | 20.20 | 79.282 | 0.007 | 0 | 0.060 |

a) AcAc = Acetylacetonate; DIPY = 2,2'-dipyridyl; BSEDA = bis (salicylidene)ethylenediamine

b) U. S. Patent 4,551,553 (ARCO)

With reference to the table, it will be noticed that the use of a soluble ruthenium catalyst promoted with an amine ligand resulted in an enhanced rate of decomposition of the tertiary butyl hydroperoxide as shown by the increased conversion for the given reaction time and an enhanced selectivity of the tertiary butyl hydroperoxide to tertiary butyl alcohol as compared with the use of a soluble ruthenium catalyst without an amine ligand or a binary mixture of a ruthenium compound with a chromium compound.

## USE OF PROMOTED METAL PHTHALOCYANINES

Metal phthalocyanine catalysts, such as a metal phthalocyanines of the type disclosed in William et al. U.S. Patent No. 3816548, can be used in the method of the present invention, if they are promoted with a

promoter selected from

(a) an amine
(b) a rhenium compound;
(c) a free radical inhibitor and/or a mercaptan;
(d) a base, and
(e) a metal borate promotor.

The metal phthalocyanine catalyst used in this invention is suitably a phthalocyanine of a heavy metal selected from the Group IB, Group VIIB, or Group VIIIB of the Periodic Table.

Phthalocyanine itself is:

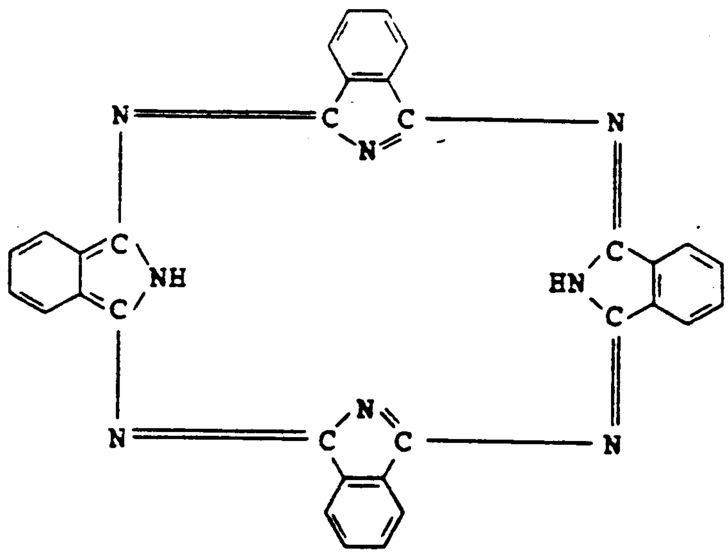

The two hydrogens in the center of the molecule are replaced by metals from these groups. The metals may be in a high oxidation state or a lower oxidation state. For example, Ferric ($Fe^{+++}$) or Ferrous ($Fe^{++}$) may be used. In addition, from 1 to 16 of the peripheral hydrogen atoms on the 4 benzene rings may be replaced with halogen atoms and by numerous organic and inorganic groups. Suitable phthalocyanines include cobalt phthalocyanine, copper phthalocyanine, chromium phthalocyanine, chloroferric phthalocyanine, ferrous phthalocyanine, manganese phthalocyanine, and ruthenium phthalocyanine.

(A) A METAL PHTHALOCYANINE PROMOTED WITH AN AMINE

The solvent to be used in practicing the process may be any suitable organic solvent in which tertiary butyl hydroperoxide is soluble at least to an extent sufficient to provide a solution containing from 5 to 30 wt.% of tertiary butyl hydroperoxide. A preferred solvent is isobutane and a still more preferred solvent is tertiary butyl alcohol or a mixture thereof with isobutane. In accordance with the more preferred embodiment the charge material for the process will comprise a 5 to a 30 wt.% solution of tertiary butyl hydroperoxide and tertiary butyl alcohol.

The process may be batchwise in kettles or by continuously passing the reactants through a reactor.

In any event, the solvent solution of tertiary butyl hydroperoxide that is charged to the reaction zone will have dissolved or slurried therein from 0.001 to 5 wt.%, based on the weight of the tertiary butyl hydroperoxide, of a metal phthalocyanine catalyst and from 0.2 to 5 parts by weight, per part of metal phthalocyanine catalyst, of an amine, specifically of imidazole.

The catalytic decomposition of the tertiary butyl hydroperoxide is preferably conducted at a temperature within the range of about 20° to about 120°C. and, more preferably, at a temperature within the range of 30 to 60°C. The reaction is preferably conducted at autogeneous pressure although superatmospheric pressures up to about 1000 psig. (6900 kPa g.p.) may be used, if desired.

Flow rates of the charge solution to the reaction zone should be adjusted in order to provide an appropriate contact time within the reactor. In a batch process, the holding time may suitably be from 0.5 to 10 hours.

In accordance with the more preferred embodiment isobutane is reacted with oxygen in an oxidation zone under oxidation reaction conditions including a temperature of 135° to 155°C., a pressure of 300 to

800 psig. (2070 to 5520 kPa g.p.), and a holding time of 2 to 6 hours to provide an initial oxidation reaction product comprising unreacted isobutane, tertiary butyl hydroperoxide, and some tertiary butyl alcohol The oxidation reaction product is fractionated in any appropriate manner (e.g., by distillation in a distillation zone) to remove the isobutane therefrom for recycle and to provide a solution of tertiary butyl hydroperoxide and tertiary butyl alcohol which will normally contain from 5 to 30 wt.% of tertiary butyl hydroperoxide. If the tertiary butyl hydroperoxide concentration is excessive, additional tertiary butyl alcohol may be added.

The solvent solution of tertiary butyl alcohol in organic solvents (e.g., tertiary butyl alcohol solvent solution of tertiary butyl hydroperoxide) is then charged to a catalytic hydroperoxide decomposition zone where it is brought into contact with an imidazole-promoted metal phthalocyanine catalyst to substantially selectively convert the tertiary butyl hydroperoxide to tertiary butyl alcohol with high yields and selectivities.

As indicated, the catalytic decomposition of the tertiary butyl hydroperoxide in the catalytic hydroperoxide decomposition reaction zone may suitably be conducted at a temperature within the range of 20° to 120°C. (and more preferably from 30° to 60°C.) at autogeneous pressure or if desired at a superatmospheric pressure up to 1000 psig. (6900 kPa g.p.) for a contact time within the range of 0.5 to 10 hours.

The reaction product from the tertiary butyl hydroperoxide decomposition step may then be fractionated in any suitable manner, such as, for example, in the manner shown in the process disclosed in above identified copending U. S. patent application S.N. 06/945,628 filed December 23, 1986. In accordance with a process recovery sequence of this nature, both the tertiary butyl alcohol and the ditertiary butyl peroxide will be recovered in purified form as products of the reaction.

Alternately, a crude tertiary butyl alcohol product stream contaminanted with ditertiary butyl peroxide and other contaminants may be obtained which will then be further treated either thermally, in accordance with the process of the Grane et al. U. S. patents, or catalytically by one of the processes disclosed in the copending Sanderson et al. patent applications to convert the ditertiary butyl peroxide to tertiary butyl alcohol and to otherwise significantly reduce the level of contamination of the other oxygen-containing impurities.

SPECIFIC EXAMPLES (USE OF AMINE)

The invention will be further illustrated by the following specific examples which are given by way of illustration and not as a limitation of this invention.

Procedure: Tube Experiments

A 150-ml Fisher-Porter pressure tube equipped with pressure gauge, rupture disk, and shut-off valve was charged with 15.0g of a 20% TBHP solution in TBA, and catalyst(s). The tube was suspended in a constant temperature bath (+ or - 0.2°C.) for the desired period of time at the required temperature. The tube was shaken from time to time during the run. At the end of the run, the tube was placed in cold water (15-20°C.) for 15 minutes. The pressure was then slowly released. The contents were analyzed by GC. The results are shown in the attached Table I.

Procedure: Autoclave Experiment

A 1000 cc stainless-steel autoclave equipped with Magne-drive stirrer, cooling coils, electric heater, etc., was charged with 300.0g 20% TBHP in TBA along with catalyst. The mixture was then sealed, stirred at 300 rpm, and heated to the desired temperature for the required time. The mixture was then cooled to ambient temperature, slowly vented, and the contents transferred to a tared container. The liquid contents were analyzed by GC.

TABLE I

Decomposition of 20% TBHP (in TBA) in the Presence of Various Phthalocyanines Plus Imidazole

| N.B. No. | Catalyst(s)[a] | Time (Hr) | Temp (°C) | Reactor[b] | TBHP% | Wt.% TBA | Wt.% Acetone | Wt.% MeOH | Wt.% DTBP |
|---|---|---|---|---|---|---|---|---|---|
| 6261 | | | | | | | | | |
| -15 | Mn(II)PCY[.30g] | 4.0 | 40.0 | C | 5.528 | 91.808 | 0.312 | <0.1 | 2.052 |
| -24 | Mn(II)PCY[.30g] IM [1.0g] | 4.0 | 40.0 | C | 0.891 | 95.413 | 0.658 | <0.1 | 2.585 |
| -28 | Fe(III)PCYCl [.01g] | 0.5 | 40.0 | T | 1.882 | 94.886 | 0.558 | <0.1 | 2.249 |
| -30 | Fe(III)PCYCl [.01g] IM [.01g] | 0.5 | 40.0 | T | 3.439 | 93.587 | 0.435 | <0.1 | 2.098 |
| -31 | Mn(II)PCY[.30g] IM [.50g] | 4.0 | 40.0 | C | 2.774 | 93.936 | 0.450 | <0.1 | 2.415 |
| -32 | Fe(III)PCYCl [.01g] IM [.01g] | 1.0 | 40.0 | T | 0.656 | 95.767 | 0.677 | <0.1 | 2.243 |
| -25 | Fe(III)PCYCl [.01g] | 1.0 | 40.0 | T | 1.882 | 94.962 | 0.519 | <0.1 | 2.290 |
| -33 | VOPCY [.01g] | 7.0 | 40.0 | T | N.R.[c] | - | - | - | - |
| -34 | VOPCY [.01g] IM [.01g] | 5.0 | 40.0 | T | N.R.[c] | - | - | - | - |
| -36 | Mn(II) PCY [.30g] IM [.70g] | 4.0 | 40.0 | C | 1.282 | 95.189 | 0.540 | <0.1 | 2.515 |
| | Starting Material | - | - | - | 20.345 | 79.345 | 0 | 0 | 0.045 |

a) PCY = phthalocyanine
IM = imidazole

b) T = 150 cc Fisher-Porter pressure tube, 15.0g scale
C = 1000 cc stainless steel autoclave, 300 g scale

c) N.R. = no detectable reaction

With reference to the table, it will be noticed that the use of imidazole as a promoter resulted in an enhanced rate of decomposition of the tertiary butyl hydroperoxide as shown by the increased conversion for the given reaction time and an enhanced selectivity of the tertiary butyl hydroperoxide to tertiary butyl alcohol.

(B) A METAL PHTHALOCYANINE PROMOTED WITH A RHENIUM COMPOUND

The rhenium compound may suitably be an organic or inorganic rhenium compound at least partially soluble in the reaction medium such as rhenium heptoxide-p-dioxane, ammonium rhenium tetroxide, rhenium decacarbonyl, oxotrichloro-bis-(triphenylphosphine) rhenium V, etc.

EXAMPLES (PROMOTION WITH RHENIUM COMPOUND)

In general, 0.2 to 1 part by weight of rhenium compound should be used for each part of phthalocyanine catalyst.

The solvent to be used in practicing the process may be any suitable organic solvent in which tertiary butyl hydroperoxide is soluble at least to an extent sufficient to provide a solution containing from 0.1 to 80 wt.% of tertiary butyl hydroperoxide. A preferred solvent is isobutane and a still more preferred solvent is tertiary butyl alcohol or a mixture thereof with isobutane. In accordance with the most preferred embodiment the charge material for the process will comprise 5 to a 50 wt.% solution of tertiary butyl hydroperoxide in tertiary butyl alcohol.

The process of the present invention may be conducted batchwise in kettles or by continuously passing the reactants through a reactor.

In any event, the solvent solution of tertiary butyl hydroperoxide that is charged to the reaction zone will also have dissolved or slurried therein from 0.001 to 5 wt.%, based on the weight of the tertiary butyl hydroperoxide, of a metal phthalocyanine catalyst and from 0.2 to 1 part by weight, per part of metal phthalocyanine catalyst, of a rhenium compound.

The catalytic decomposition of the tertiary butyl hydroperoxide is preferably conducted at a temperature within the range of 20° to 80°C. and, more preferably, at a temperature within the range of 20 to 40°C. The reaction is preferably conducted at autogeneous pressure although superatmospheric pressures up to about 1000 psig. (6900 kPa g.p.) may be used, if desired.

Flow rates of the charge solution to the reaction zone should be adjusted in order to provide an appropriate contact time within the reactor. In a batch process, the holding time may suitably be from 0.5 to 10 hours.

In accordance with the most preferred embodiment of the present invention, isobutane is reacted with oxygen in an oxidation zone under oxidation reaction conditions including a temperature of 135° to 155°C., a pressure of 300 to 800 psig. (2070 to 5520 kPa g.p.) and a holding time of 0.5 to 5 hours to provide an initial oxidation reaction product comprising unreacted isobutane, tertiary butyl hydroperoxide, and some tertiary butyl alcohol. The oxidation reaction product is fractionated in any appropriate manner (e.g., by distillation in a distillation zone) to remove the isobutane therefrom for recycle and to provide a solution of tertiary butyl hydroperoxide and tertiary butyl alcohol which will normally contain from 5 to 30 wt.% of tertiary butyl hydroperoxide. If the tertiary butyl hydroperoxide concentration is excessive, additional tertiary butyl alcohol may be added.

The solvent solution of tertiary butyl alcohol in organic solvents (e.g., tertiary butyl alcohol solvent solution of tertiary butyl hydroperoxide) is then charged to a catalytic hydroperoxide decomposition zone where it is brought into contact with a rhenium-promoted metal phthalocyanine catalyst to substantially selectively convert the tertiary butyl hydroperoxide to tertiary butyl alcohol with high yields and selectivities.

The reaction product from the tertiary butyl hydroperoxide decomposition step may then be fractionated in any suitable manner, such as, for example, in the manner shown in the process disclosed in above identified copending U. S. patent application S.N. 06/945,628 filed December 23, 1986. In accordance with a process recovery sequence of this nature, both the tertiary butyl alcohol and the ditertiary butyl peroxide will be recovered in purified form as products of the reaction.

Alternately, a crude tertiary butyl alcohol product stream contaminanted with ditertiary butyl peroxide and other contaminants may be obtained which will then be further treated either thermally, in accordance with the process of the Grane et al. U. S. patents, or catalytically by one of the processes disclosed in the copending Sanderson et al. patent applications to convert the ditertiary butyl peroxide to tertiary butyl alcohol and to otherwise significantly reduce the level of contamination of the other oxygen-containing impurities.

Thus the invention further relates to a solution of t-butyl hydroxide in t-butyl alcohol which contains from 5 to 30 wt.% of butyl hydroperoxide wherein unreacted isobutane is continuously separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that it comprises:

(a) 0.001 to 5 wt % based on the weight of the t-butyl hydroperoxide of a soluble rhenium catalyst compound and 1 to 3 parts by weight, based on the weight of the rhenium compound, of a bidentate ligand selected from 2,2'-dipyridyl and bis(salicylidene)ethylenediamine.

(b) conducting said hydroperoxide decomposition reaction in the presence of said thus-prepared

hydroperoxide decomposition catalyst in said hydroperoxide decomposition zone in liquid phase with agitation under reaction conditions including a temperature within the range of 30° to about 80°C. and autogeneous pressure, and

(c) continuously removing a stream of said hydroperoxide conversion product from said hydroperoxide conversion zone.

(d) said bidentate ligand having the formula as previously defined.

The invention further relates to a continuous method for preparing t - butyl alcohol wherein

(a) the oxidation reaction conditions include a temperature within the range of 135° to 155°C, a pressure of 300 to 800 psig. (2070 to 5520 kPa g.p.) and a holding time of 2 to 6 hours to provide an initial reaction mixture comprising unreacted isobutane and isobutane oxidation reaction products, principally t-butyl hydroperoxide and t-butyl alcohol,

(b) continuously separating unreacted isobutane from said initial reaction mixture to provide a charge stock,

(c) continuously feeding said charge stock to a hydroperoxide decomposition zone, adding said decomposition catalyst, and

(d) continuously conducting a catalytic hydroperoxide decomposition reaction in said hydroperoxide decomposition zone in liquid phase with agitation under reaction conditions at a temperature within the range of 30° to 80°C. and autogenous pressure, to convert said t-butyl hydroperoxide to decomposition products, principally t-butyl alcohol, to provide a hydroperoxide conversion product.

SPECIFIC EXAMPLES (PROMOTION WITH RHENIUM COMPOUND)

The invention will be further illustrated by the following specific examples which are given by way of illustration and not as a limitation of this invention.

Procedure: Tube Experiments

A 150-ml Fisher-Porter pressure tube equipped with pressure gauge, rupture disk, and shut-off valve was charged with 15.0g of a 18.43% TBHP solution in TBA, and catalyst(s). The tube was suspended in a constant temperature bath (+ or - 0.2°C.) for the desired period of time at the required temperature. The tube was shaken from time to time during the run. At the end of the run, the tube was placed in cold water (15-20°C.) for 15 minutes. The pressure was then slowly released. The contents were analyzed by GC. The results are shown in the attached Table I.

TABLE I

Decomposition of tert-Butyl Hydroperoxide to tert-Butyl Alcohol

| N.B. Number | Catalyst(s)[a] | Time (Hr) | Temp (°C) | TBHP | Wt.% TBA | Acetone | MeOH | DTBP |
|---|---|---|---|---|---|---|---|---|
| 6261-76 | Co(II)PCY [.01g] | 2.0 | 60.0 | 12.40 | 86.37 | 0.16 | <0.1 | 0.63 |
| 6261-80 | Co(II)PCY [.01g] $ReO_7$.3Diox [.01g] | 2.0 | 60.0 | 7.28 | 90.60 | 0.28 | 0.17 | 1.21 |
| 6261-84 | Co(II)SEDA [.01g] | 2.0 | 60.0 | 18.22 | 81.23 | 0.27 | <0.1 | 0.11 |
| 6261-83 | Co(II)SEDA [.01g] $Re_2O_7$3Diox [.01g] | 2.0 | 60.0 | 17.58 | 80.01 | 0.26 | 0.63 | 1.23 |
| 6261-81 | $Re_2O_7$3Diox [.01g] | 2.0 | 60.0 | 16.83 | 79.05 | 0.27 | 0.37 | 2.12 |
| 6261-82 | Fe(III)PCYCl [.01g] | 2.0 | 60.0 | 0.05 | 96.45 | 1.32 | 0.10 | 1.46 |
| 6261-87 | Fe(III)PCYCl [.01g] | 1.0 | 25.0 | 5.65 | 91.48 | 0.23 | <0.1 | 2.42 |
| 6261-88 | Fe(III)PCYCl [.01g] $Re_2O_7$.3Diox [.008g] | 1.0 | 25.0 | 0.023 | 95.83 | 0.64 | <0.1 | 3.18 |
| 6261-89 | Fe(III)PCYCl[.0075g] $Re_2O_7$.3Diox[.0025g] | 0.25 | 25.0 | 0.28 | 95.55 | 0.77 | <0.1 | 3.09 |
| 6261-90 | Fe(III)PCYCl [.00375g] $Re_2O_7$.3Diox[.00125g] | 1.0 | 25.0 | 0.046 | 95.70 | 0.63 | <0.1 | 3.26 |
| 6261-93 | Fe(III)PCYCl [.00375g] $Re_2O_7$.3Diox[.00125g] | 0.50 | 25.0 | 0.33 | 95.70 | 0.55 | <0.1 | 3.09 |
| 6261-92 | Fe(III)PCYCl [.01g] $NH_4ReO_4$ [.01g] | 0.50 | 25.0 | 2.93 | 93.72 | 0.60 | <0.1 | 2.50 |
| 6261-97 | Fe(III)PCYCl [.01g] $Re_2(CO)_{10}$ [.01g] | 2.0 | 25.0 | 1.34 | 94.52 | 0.62 | <0.1 | 3.22 |
| — | Starting Material | | | 18.43 | 81.02 | ~0 | ~0 | 0.06 |

(a) PCY = phthalocyanine; Diox = Dioxane; SEDA = bis(salicylidene)ethylenediamino

Discussion of Results in Table

In experiment 6261-76, cobalt phthalocyanine is shown to decompose TBHP to TBA with 12.4% TBHP remaining at 60.0°C. (2.0 hr. reaction time). In the presence of cobalt phthalocyanine and a rhenium complex [6261-80], the TBHP remaining is only 7.28 wt.%

In the presence of another cobalt (II) complex [bis(salicylidene)ethylenediamino cobalt(II)] the conversion is less than 10% [6261-84] even in the presence of the rhenium complex [see 6261-83]. These experiments show that it is necessary to have both a phthalocyanine compound and a rhenium compound to obtain good results.

Iron (III) phthalocyanine is more active than the cobalt (II) phthalocyanine and only 0.05% TBHP remains at 60 °C. and 2.0 hr. reaction time [6261-82]. At lower temperatures (25 °C., 1.0 hr. reaction time), a 5.65% TBHP remains [6261-87]. Under the same conditions but with an added rhenium complex, the conversion is >99% with no loss in selectivity [6261-88]. Various ratios of Fe to Re and Fe + Re to TBHP were also studied [see 6261-89, 90, 93].

The last two entries in the table show that other rhenium compounds are also effective catalysts [see 6261-92 and 6261-97]. These two catalysts ($NH_4ReO_4$ and $Re_2(CO)_{10}$) were not as active as $Re_2O_7$.3Diox.

Having thus described our invention, what is claimed is:

(C) A METAL PHTHALOCYANINE CATALYST PROMOTED WITH A MERCAPTAN AND A FREE RADICAL INHIBITOR

The solvent to be used in practicing may be any suitable organic solvent in which tertiary butyl hydroperoxide is soluble at least to an extent sufficient to provide a solution containing from 5 to 50 wt.% of tertiary butyl hydroperoxide. A preferred solvent is isobutane and a still more preferred solvent is tertiary butyl alcohol or a mixture thereof with isobutane. In accordance with the more preferred embodiment the charge material for the process will comprise a 5 to a 30 wt.% solution of tertiary butyl hydroperoxide and tertiary butyl alcohol.

The yield and selectivity are improved if the catalyst composition also contains an appropriate mercaptan and/or an appropriate free radical inhibitor. Mercaptans and free radical inhibitors are well known classes of catalyst modifiers that are discussed in greater detail, for example, in texts such as W.A. Pryor "Free Radicals", McGraw Hill, New York, 1966.

A free-radical inhibitor is a compound which will scavenge free-radicals. These inhibitors may react with radicals to produce another radical either too stable to react or reactive in some new pathway. Phenols, amines, quinones and other similar materials with active hydrogen are inhibitors.

Examples of inhibitors are: phenol, hydroquinone, 2,3-dihydroxynapthalene, quinone, N,N′-diphenylphenylenedi-amine, etc.

An alkyl thiol or alkyl mercaptan (R-S-H) where R = alkyl group with $C_1$ to $C_{18}$. For example, methane thiol (methyl mercaptan), butane thiol, octane thiol, dodecane thiol (dodecyl mercaptan).

A catalytically effective amount of a metal phthalocyanine should be used (e.g., from 0.001 to 5 wt.%, based on the weight of the tertiary butyl hydroperoxide charge. From 0.2 to 5 parts by weight of chain transfer agent per part of chloroferric phthalocyanine may be used, as may from 0.2 to 5 parts by weight of mercaptan, per part of chloroferric phthalocyanine catalyst.

The present invention provides a continuous method for preparing t-butyl alcohol from a reaction mixture comprising a solution of t-butyl hydroperoxide in t-butyl alcohol with 2 to 90 wt.%. of t-butly hydroperoxide wherein unreacted isobutane is continuously separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that it comprises:

(a) adding from 0.001 to 5 wt.%, based on the weight of the t-butyl hydroperoxide of chloroferric phthalocyanine, 1 to 1.5 parts by weight, per part of the chloroferric phthalocyanine of 2,3-dihydroxynaphthalene and 1 to 20 parts by weight, per part of 2,3-dihydroxynaphthalene of a $C_1$-$C_{18}$ alkyl thiol to said charge stock as said hydroperoxide decomposition catalyst,

(b) conducting said hydroperoxide decomposition reaction in the presence of said thus-prepared hydroperoxide decomposition catalyst in said hydroperoxide decomposition zone in liquid phase with agitation under reaction conditions including a temperature within the range of 20° to 125 °C. and autogenous pressure, and

(c) continuously removing a stream of said hydroperoxide conversion product from said hydroperoxide conversion zone.

The reaction mixture may comprise a solution of t-butyl hydroperoxide in t-butyl alcohol which contains from 20 to 50 wt.% of t-butyl hydroperoxide together with 1 to 6 parts by weight, per part of the chloroferric phthalocyanine of a lithium, sodium, magnesium, zinc, or calcium borate to said charge stock as said hydroperoxide decomposition catalyst.

## EXAMPLES

The process of the present invention may be conducted batchwise in kettles or by continuously passing the reactants through a reactor.

The solvent solution of tertiary butyl hydroperoxide that is charged to the reaction zone should also have dissolved or slurried therein from 0.001 to 5 wt.%, based on the weight of the tertiary butyl hydroperoxide, of a metal phthalocyanine catalyst, from 0.2 to 5 parts by weight, per part of phthalocyanine catalyst, of a mercaptan and 0.2 to 5 parts by weight of a free radical inhibitor per part of phthalocyanine catalyst.

The catalytic decomposition of the tertiary butyl hydroperoxide is preferably conducted at a temperature within the range of 20° to 120°C. and, more preferably, at a temperature within the range of 30 to 60°C. The reaction is preferably conducted at autogeneous pressure although superatmospheric pressures up to about 1000 psig. (6900 kPa g.p.) may be used, if desired.

Flow rates of the charge solution to the reaction zone should be adjusted in order to provide an appropriate contact time within the reactor. In a batch process, the holding time may suitably be from 0.5 to 10 hours.

In accordance with the most preferred embodiment of the present invention, isobutane is reacted with oxygen in an oxidation zone under oxidation reaction conditions including a temperature of 135° to 155°C., a pressure of 300 to 800 psig., (2070 to 5520 kPa g.p) and a holding time of 0.5 to 5 hours to provide an initial oxidation reaction product comprising unreacted isobutane, tertiary butyl hydroperoxide, and some tertiary butyl alcohol. The oxidation reaction product is fractionated in any appropriate manner (e.g., by distillation in a distillation zone) to remove the isobutane therefrom for recycle and to provide a solution of tertiary butyl hydroperoxide and tertiary butyl alcohol which will normally contain from 5 to 30 wt.% of tertiary butyl hydroperoxide. If the tertiary butyl hydroperoxide concentration is excessive, additional tertiary butyl alcohol may be added.

The solvent solution of tertiary butyl alcohol in organic solvents (e.g., tertiary butyl alcohol solvent solution of tertiary butyl hydroperoxide) is then charged to a catalytic hydroperoxide decomposition zone where it is brought into contact with a metal phthalocyanine catalyst, a chain transfer agent and a free radical inhibitor to substantially selectively convert the tertiary butyl hydroperoxide to tertiary butyl alcohol with high yields and selectivities.

The reaction product from the tertiary butyl hydroperoxide decomposition step may then be fractionated in any suitable manner, such as, for example, in the manner shown in the process disclosed in above identified copending U. S. patent application S.N. 06/945,628 filed December 23, 1986. In accordance with a process recovery sequence of this nature, both the tertiary butyl alcohol and the ditertiary butyl peroxide will be recovered in purified form as products of the reaction.

Alternately, a crude tertiary butyl alcohol product stream contaminanted with ditertiary butyl peroxide and other contaminants may be obtained which will then be further treated either thermally, in accordance with the process of the Grane et al. U. S. patents, or catalytically by one of the processes disclosed in the copending Sanderson et al. patent applications to convert the ditertiary butyl peroxide to tertiary butyl alcohol and to otherwise significantly reduce the level of contamination of the other oxygencontaining impurities.

## SPECIFIC EXAMPLES (PROMOTION WITH MERCAPTAN and a free radical inhibitor)

The invention will be further illustrated by the following specific examples which are given by way of illustration and not as a limitation of this invention.

### Procedure

A 100 ml three-necked flask equipped with a water-cooled condenser, thermometer and magnetic stirrer was suspended in a water bath. The flask was charged with the catalyst(s) shown in the table and then 20.0g of 18.43% TBHP in TBA. The contents were vigorously stirred and 1 ml aliquots withdrawn at the times indicated. Analysis was by GC.

TABLE I

Decomposition of TBHP to TBA

| Notebook Number | Time (Hr) | Temp (°C) | FePCYCl[a] (g) | 2,3-DiHy[b] Napth (g) | DT[c] (g) | Weight % TBHP | TBA | Acetone | MeOH | DTBP | Exotherm |
|---|---|---|---|---|---|---|---|---|---|---|---|
| 6274-67 | 1.0 | 30.0 | 0.05 | 0 | 2.54 | 0.034 | 93.03 | 0.94 | 2.26 | 0.88 | Yes |
| 6274-70 | 4.0 | 26.0 | 0.05 | 0.10 | 0 | 2.10 | 94.00 | 0.52 | <0.1 | 0.21 | No |
| 6274-72 | 1.0 | 26.0 | 0.05 | 0.05 | 0 | 0.36 | 95.12 | 0.66 | <0.1 | 3.36 | No |
| 6274-74 | 0.5 | 33.0 | 0.05 | 0.05 | 0.84 | 0.39 | 94.81 | 1.10 | 0.81 | 1.30 | Yes |
| 6274-75 | 0.5 | 33.0 | 0.05 | 0.05 | 0.14 | 0.06 | 96.11 | 0.97 | 0.05 | 2.15 | Yes |
| 6274-76 | 0.5 | 31.0 | 0.05 | 0.07 | 0.07 | 0.14 | 95.97 | 0.79 | <0.1 | 2.57 | Yes |
| 6274-77 | 0.5 | 31.0 | 0.05 | 0.20 | 0.84 | 9.34 | 88.37 | 0.34 | 0.89 | 0.73 | Yes |
| 6274-44 | 1.0 | 40.0 | 0.01 | 0 | 0 | 3.89 | 92.96 | 0.67 | <0.1 | 2.11 | Unk |
| Starting Material | | | | | | 18.43 | 81.02 | 0 | 0 | 0.06 | |

(a) Chloroferric phthalocyanine
(b) 2,3-Dihydroxynaphthalene
(c) Dodecane Thiol

Discussion of Results in Table

Experiment No.6274-74 which was conducted in the presence of Fe(III)PCYCl and dodecane thiol (DT) shows high conversion of TBHP and good selectivity to TBA. It may be compared to 6274-44 which had no

DT added. In the presence of DT, the DTBP dropped from 2.11 to 0.88. The presence of the inhibitor 2,3-dihydroxy naphthalene lowers the percent TBHP conversion but does not lower the percent DTBP formed. (See Exp. No. 6274-70 and Exp. No.6274-722). In the presence of both inhibitor and DT, the DTBP remains low. In fact, the percent DTBP formed appears to be proportional to the amount of DT added. (See Exp. No. 6274-76 and Exp. No. 6274-77).

(D) A METAL PHTHALOCYANINE PROMOTED WITH A BASE

Any suitable inorganic base having a pH greater than about 7.5 when 0.10 mole is dissolved in 1 l. of water may be used, such as an alkali metal carbonate, bicarbonate, acetate and/or hydrogen phosphate, formate, benzoate, etc. Examples of suitable bases include compounds such as sodium acetate, sodium bicarbonate, sodium carbonate, lithium carbonate, lithium bicarbonate, sodium hydrogen phosphate, sodium formate, potassium benzoate, potassium acetate. 1 to 5 parts by weight of base should be used for each part of metal phthalocyanine catalyst.

The solvent to be used in practicing the process may be any suitable organic solvent in which tertiary butyl hydroperoxide is soluble at least to an extent sufficient to provide a solution containing from 5 to 50 wt.% of tertiary butyl hydroperoxide. A preferred solvent is isobutane and a still more preferred solvent is tertiary butyl alcohol. In accordance with the more preferred embodiment, the charge material for the process will comprise a 5 to a 30 wt.% solution of tertiary butyl hydroperoxide and tertiary butyl alcohol.

The process may be conducted batchwise in kettles or by continuously passing the reactants through a reactor.

In any event, the solvent solution of tertiary butyl hydroperoxide that is charged to the reaction zone will also have dissolved or slurried therein from 0.001 to 5 wt.%, based on the weight of the tertiary butyl hydroperoxide, of a metal phthalocyanine catalyst and from 1 to 5 parts by weight, per part of metal phthalocyanine catalyst, of a suitable base.

The catalytic decomposition of the tertiary butyl hydroperoxide is preferably conducted at a temperature within the range of 20° to 125°C. and more preferably, at a temperature within the range of 20 to 50°C. The reaction is preferably conducted at autogeneous pressure although superatmospheric pressures up to about 1000 psig. (6900 kPa g.p.) may be used, if desired.

Flow rates of the charge solution to the reaction zone should be adjusted in order to provide an appropriate contact time within the reactor. In a batch process, the holding time may suitably be from 0.5 to 10 hours.

In accordance with the most preferred embodiment isobutane is reacted with oxygen in an oxidation zone under oxidation reaction conditions including a temperature of 135° to 155°C., a pressure of 300 to 800 psig., (2070 to 5520 kPa g.p) and a holding time of 0.5 to 5 hours to provide an initial oxidation reaction product comprising unreacted isobutane, tertiary butyl hydroperoxide, and some tertiary butyl alcohol. The oxidation reaction product is fractionated in any appropriate manner (e.g., by distillation in a distillation zone) to remove the isobutane therefrom for recycle and to provide a solution of tertiary butyl hydroperoxide and tertiary butyl alcohol which will normally contain from 5 to 30 wt.% of tertiary butyl hydroperoxide. If the tertiary butyl hydroperoxide concentration is excessive, additional tertiary butyl alcohol may be added.

The solvent solution of tertiary butyl alcohol in organic solvents (e.g., tertiary butyl alcohol solvent solution of tertiary butyl hydroperoxide) is then charged to a catalytic hydroperoxide decomposition zone where it is brought into contact with a base-promoted metal phthalocyanine catalyst to substantially selectively convert the tertiary butyl hydroperoxide to tertiary butyl alcohol with high yields and selectivities.

The reaction product from the tertiary butyl hydroperoxide decomposition step may then be fractionated in any suitable manner, such as, for example, in the manner shown in the process disclosed in above identified copending U. S. patent application S.N. 06/945,628 filed December 23, 1986. In accordance with a process recovery sequence of this nature, both the tertiary butyl alcohol and the ditertiary butyl peroxide will be recovered in purified form as products of the reaction.

Alternately, a crude tertiary butyl alcohol product stream contaminanted with ditertiary butyl peroxide and other contaminants may be obtained which will then be further treated either thermally, in accordance with the process of the Grane et al. U. S. patents, or catalytically by one of the processes disclosed in the copending Sanderson et al. patent applications to convert the ditertiary butyl peroxide to tertiary butyl alcohol and to otherwise significantly reduce the level of contamination of the other oxygencontaining impurities.

SPECIFIC EXAMPLES (PROMOTION WITH A BASE)

The invention will be further illustrated by the following specific examples which are given by way of illustration and not as a limitation of this invention.

Procedure: Tube Experiments

A 150-ml Fisher-Porter pressure tube equipped with pressure gauge, rupture disk, and shut-off valve was charged with 15.0g of a 18.43% TBHP solution in TBA, and catalyst(s). The tube was suspended in a constant temperature bath (+ or - 0.2° C.) for the desired period of time at the required temperature. The tube was shaken from time to time during the run. At the end of the run, the tube was placed in cold water (15-20° C.) for 15 minutes. The pressure was then slowly released. The contents were analyzed by GC. The results are shown in the attached Table I.

TABLE I

Decomposition of TBHP (in TBA) in the Presence of Iron Phthalocyanines and a Base[a]

| N.B. Number | Catalyst(s)[b] | Time (Hr) | Temp (°C) | TBHP | Products, Wt.%[c,d] TBA | Acetone | MeOH | DTBP |
|---|---|---|---|---|---|---|---|---|
| 6274-10 | Fe(III)PCYCl[.01g] | 1.0 | 40.0 | 3.65 | 93.59 | 0.31 | <0.1 | 2.12 |
| 6274-13 | Fe(III)PCYCl[.01g] $NaHCO_3$[.01g] | 1.0 | 40.0 | 2.27 | 94.53 | 0.63 | <0.1 | 2.23 |
| 6274-15 | Fe(III)PCY[.01g] $NaHCO_3$[.03g] | 1.0 | 40.0 | 0.39 | 96.28 | 0.70 | <0.1 | 2.20 |
| 6274-14 | Fe(III)PCY[.01g] NaOAc[.02g] | 1.0 | 40.0 | 1.08 | 95.52 | 0.74 | <0.1 | 2.26 |
| 6274-16 | Fe(III)PCY[.01g] NaOAc[.04g] | 1.0 | 40.0 | 0.61 | 96.09 | 0.57 | <0.1 | 2.29 |
| 6274-19 | Fe(III)PCY[.01g] $Na_4P_2O_7$[.03g] | 1.2 | 40.0 | 1.02 | 95.51 | 0.74 | <0.1 | 2.32 |
| 6274-20 | $NH_4H_2PO_4$[.03g] Fe(III)PCY[.01g] | 1.0 | 40.0 | 2.35 | 94.41 | 0.62 | <0.1 | 2.25 |
| 6274-21 | Fe(III)PCY[.01g] $(NH_4)_2HPO_4$[.03g] | 1.0 | 40.0 | 3.45 | 93.52 | 0.56 | <0.1 | 2.14 |
| 6274-25 | Fe(III)PCY[.01g] $Li_2CO_3$[.02g] | 1.0 | 40.0 | 0.93 | 95.81 | 0.56 | <0.1 | 2.18 |
| 6274-18 | NaOAc [.03g] | 1.0 | 40.0 | 18.28 | 80.91 | 0.53 | <0.1 | 0.089 |
| 6274-17 | $NaHCO_3$ [.02g] | 1.0 | 40.0 | 18.41 | 81.04 | ~0 | <0.1 | 0.086 |
| — | Starting Material | | | 18.43 | 81.02 | ~0 | ~0 | 0.06 |

(a) The initial TBHP concentration was 18.34%; remainder mostly TBA. 15.0g charged.
(b) PCY = Phthalocyanine
(c) Determined by GC (Hewlett-Packard 5890, 3392H Integrator)
(d) Fused silica capillary column; 5% methyl phenyl silicone oil; 0.53 mm ID, 3.0μ film; Prog. 35-165°C.

Chloroferric phthalocyanine catalyzes the decomposition of TBHP to TBA. In one hour at 40 ° C., 3.65% of the TBHP remains (N.B. No. 6274-10). In the presence of $NaHCO_3$, only 0.39% of the TBHP remains. Selectivity to TBA is high (6274-15). The same trend is seen with most other salts tried including NaOAc, $Li_2CO_3$, $Na_4P_2O_7$, etc. Several control experiments were also conducted. Both NaOAc and $NaHCO_3$ (under the same reaction conditions) showed no TBHP decomposition (6274-17 and 6274-18).

(E) A METAL PHTHALOCYANINE PROMOTED WITH A METAL BORATE

Any appropriate metal borate promoter may be used, such as an alkali metal borate, an alkaline earth

metal borate, a borate of a Group IIB metal. Examples of appropriate metal borates include compounds such as sodium borate, lithium borate, magnesium borate, zinc borate, calcium borate, barium borate, sodium metaborate, lithium metaborate, ammonium borate. Mixtures of two or more such metal borates may be used, if desired.

The solvent to be used in practicing the process of any suitable organic solvent in which tertiary butyl hydroperoxide is soluble at least to an extent sufficient to provide a solution containing from 2 to 90 wt.% of tertiary butyl hydroperoxide. A preferred solvent is isobutane and a still more preferred solvent is tertiary butyl alcohol. In accordance with the most preferred embodiment, the charge material for the process will comprise a 5 to a 30 wt.% solution of tertiary butyl hydroperoxide and tertiary butyl alcohol.

## EXAMPLES

The process of the present invention may be conducted batchwise in kettles or by continuously passing the reactants through a reactor.

In any event, the solvent solution of tertiary butyl hydroperoxide that is charged to the reaction zone will also have dissolved or slurried therein from 0.001 to 5 wt.%, based on the weight of the tertiary butyl hydroperoxide, of a metal phthalocyanine catalyst and from 1 to 6 parts by weight of metal borate promoter, per part of chloroferric phthalocyanine catalyst.

The catalytic decomposition of the tertiary butyl hydroperoxide is preferably conducted at a temperature within the range of 20° to 125°C. and, more preferably, at a temperature within the range of 20 to 60°C. The reaction is preferably conducted at autogeneous pressure although superatmospheric pressures up to 1000 psig. (6900 kPa g.p) may be used, if desired.

Flow rates of the charge solution to the reaction zone should be adjusted in order to provide an appropriate contact time within the reactor. In a batch process, the holding time may suitably be from 0.5 to 10 hours.

In accordance with the most preferred embodiment of the present invention, isobutane is reacted with oxygen in an oxidation zone under oxidation reaction conditions including a temperature of 135° to 155°C., a pressure of 300 to 800 psig., (2070 to 5520 kPa g.p.) and a holding time of 0.5 to 5 hours to provide an initial oxidation reaction product comprising unreacted isobutane, tertiary butyl hydroperoxide, and some tertiary butyl alcohol. The oxidation reaction product is fractionated in any appropriate manner (e.g., by distillation in a distillation zone) to remove the isobutane therefrom for recycle and to provide a solution of tertiary butyl hydroperoxide and tertiary butyl alcohol which will normally contain from about 5 to about 30 wt.% of tertiary butyl hydroperoxide. If the tertiary butyl hydroperoxide concentration is excessive, additional tertiary butyl alcohol may be added.

The solvent solution of tertiary butyl alcohol in organic solvents (e.g., tertiary butyl alcohol solvent solution of tertiary butyl hydroperoxide) is then charged to a catalytic hydroperoxide decomposition zone where it is brought into contact with a borate-promoted metal phthalocyanine catalyst to substantially selectively convert the tertiary butyl hydropercxide to tertiary butyl alcohol with high yields and selectivities.

The reaction product from the tertiary butyl hydroperoxide decomposition step may then be fractionated in any suitable manner, such as, for example, in the manner shown in the process disclosed in above identified copending U. S. patent application S.N. 06/945,628 filed December 23, 1986. In accordance with a process recovery sequence of this nature, both the tertiary butyl alcohol and the ditertiary butyl peroxide will be recovered in purified form as products of the reaction.

Alternately, a crude tertiary butyl alcohol product stream contaminanted with ditertiary butyl peroxide and other contaminants may be obtained which will then be further treated either thermally, in accordance with the process of the Grane et al. U. S. patents, or catalytically by one of the processes disclosed in the copending Sanderson et al. patent applications to convert the ditertiary butyl peroxide to tertiary butyl alcohol and to otherwise significantly reduce the level of contamination of the other oxygencontaining impurities.

## SPECIFIC EXAMPLES (PROMOTION WITH A METAL BORATE)

The invention will be further illustrated by the following specific examples which are given by way of illustration and not as a limitation of this invention.

### Procedure: Tube Experiments

A 150-ml Fisher-Porter pressure tube equipped with pressure gauge, rupture disk, and shut-off valve

was charged with 15.0g of an 18.43% TBHP solution in TBA, and catalyst(s). The tube was suspended in a constant temperature bath (+ or - 0.2° C.) for the desired period of time at the required temperature. The tube was shaken from time to time during the run. At the end of the run, the tube was placed in cold water (15-20° C.) for 15 minutes. The pressure was then slowly released. The contents were analyzed by GC. The results are shown in the attached Table I.

TABLE I

Decomposition of tert-Butyl Hydroperoxide in the Presence of Iron Phthalocyanine and an Alkali Metal Borate[a]

| N.B. Number | Catalyst(s)[b] | Time (Hr) | Temp (°C) | TBHP | Products Wt.%[c,d] TBA | Acetone | MeOH | DTBP |
|---|---|---|---|---|---|---|---|---|
| 6274-10 | Fe(III)PCYCl[.01g] | 1.0 | 40.0 | 3.65 | 93.59 | 0.31 | <0.1 | 2.12 |
| 6274-23 | Fe(III)PCYCl[.01g] $LiBo_2$[.02g] | 1.0 | 40.0 | 0.13 | 96.31 | 0.73 | <0.1 | 2.14 |
| 6274-26 | Fe(III)PCYCl[.01g] $LiBO_2$[.01g] | 1.0 | 40.0 | 0.52 | 96.03 | 0.64 | <0.1 | 2.15 |
| 6274-41 | Fe(III)PCYCl[.01g] $LiBO_2$[.05g] | 1.0 | 40.0 | 0.17 | 96.32 | 0.57 | <0.1 | 2.20 |
| 6274-27 | Fe(III)PCYCl[.01g] $Ba(BO_2)_2$[.01g] | 1.0 | 40.0 | 0.89 | 95.65 | 0.77 | <0.1 | 2.23 |
| 6274-28 | Fe(III)PCYCl[.01g] $Zn(BO_2)_2$[.01g] | 1.0 | 40.0 | 0.54 | 96.10 | 0.65 | <0.1 | 2.22 |
| 6274-22 | Fe(III)PCYCl[.01g] $Na_2B_2O_4.8H_2O$[.02g] | 1.0 | 40.0 | 0.53 | 96.17 | 0.63 | <0.1 | 2.24 |
| 6274-24 | Fe(III)PCYCl[.01g] $Na_2B_2O_4.8H_2O$[.02g] | 1.0 | 40.0 | 0.51 | 96.15 | 0.66 | <0.1 | 2.23 |
| 6274-39 | Fe(III)PCYCl[.01g] $Na_2B_2O_4.8H_2O$[.05g] | 1.0 | 40.0 | 0.52 | 96.00 | 0.78 | <0.1 | 2.25 |
| 6274-31 | $LiBO_2$[.02g] | 1.0 | 40.0 | | No Reaction | | | |
| — | Starting Material | | | 18.43 | 81.02 | 0 | 0 | 0.06 |

(a) The initial TBHP concentration was 18.34% - remainder mostly TBA. 15.6g changed.

(b) PCY = Phthalocyanine

(c) Determined by GC (Hewlett-Packart 5890, 3392A Integrator)

(d) Fused silica capillary column, 5% methyl phenyl silicone oil, 0.53 mm ID, 3.0μ film program, 35-165°C.

Discussion of Data in Table

Chloroferric phthalocyanine alone catalyzes the decomposition of TBHP to TBA. In one hour at 40 ° C., 3.65% of the TBHP in a 15.0g initial charge remains when using 0.01g of the phthalocyanine (6274-10). Under the same conditions but with the addition of lithium borate, 0.13 to 0.17% tertiary butyl hydroperoxide remains without loss of selectivity to tertiary butyl alcohol (runs 6274-23, 6274-26 and 6274-41). Other metal borates such as barium borate, sodium borate, and zinc borate are also effective. Control experiments show that lithium borate alone does not catalyze the decomposition of TBHP under the reaction conditions (6274-31).

## Claims

**1.** A method for converting t-butyl hydroperoxide to decomposition products, principally t-butyl alcohol, by bringing a t-butyl hydroperoxide charge stock into contact with a catalytically effective amount of a catalyst in a reaction zone in liquid phase with agitation characterised in that the catalyst is a selected from:-
(a) a metal porphine compound:
(b) a soluble ruthenium catalyst compound promoted with a bidentate ligand and
(c) a promoted metal phthalocyanine compound.

**2.** A method according to Claim 1 characterised in that the bidentate ligand has the formula:

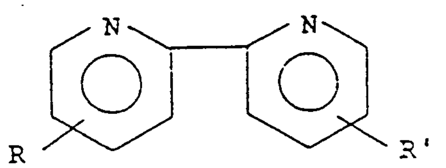

or

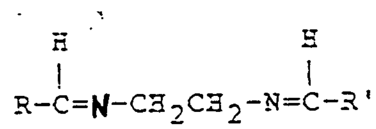

wherein R and R′ may be the same or different and are selected from hydrogen, methyl, ethyl, propyl, phenyl and chlorophenyl.

**3.** A method according to claim 1 characterised in that said metal phthalocyanine catalyst or a metal porphine compound is promoted with an amine or/and an mercaptan.

**4.** A method according to claim 1 characterised in that said metal phthalocyanine catalyst is promoted with a free radical inhibitor and/or a mercaptan, with a base or with borate, said metal being selected from Groups IB, VIIB or VIIIB of the Periodic Table.

**5.** A method according to claim 4 characterised in that the t- butyl charge stock is in solution with t-butyl alcohol.

**6.** A method according to claim 5 characterised in that the t- butyl alcohol solution consists of a soluble ruthenium catalyst and a 2,2′-dipyridyl or bis(salicylidene)ethylenediamine bidentate ligand.

**7.** A method according to claim 6 characterised in that the said soluble ruthenium catalyst compound is ruthenium acetylacetonate.

**8.** A method according to any claims 1 or 5 characterised in that said mercaptan is a $C_1$-$C_{18}$-alkyl thiol.

**9.** A method according to any of claims 1 to 5 characterised in that said amine is pyridine, quinoline, isoquinoline, imidazole or 1-alkyl or 2-alkyl imidazole wherein the alkyl group contains from 1 to 4 carbon atoms.

**10.** A method according to claim 5 characterised in that the t-butyl alcohol solution consists of a chloroferric a manganeous or a cobalt phthalocyanine catalyst and a soluble rhenium compound

selected from rhenium heptoxide-p-dioxane, ammonium rhenium tetroxide or rhenium decacarbonyl.

**11.** A method according to any of claims 1 to 5 characterised in that the said base is at least partially soluble in said t-butyl alcohol.

**12.** A method according to claim 11 characterised in that said base is an inorganic base having a pH greater than 7.5 when 0.10 mole is dissolved in 1 litre of water.

**13.** A method according to claim 11 or claim 12 characterised in that said base is an alkali metal carbonate, bicarbonate, acetate or hydrogen phosphate.

**14.** A method according to claim 13 characterised in that said base is sodium acetate, potassium acetate, sodium bicarbonate, lithium bicarbonate, lithium carbonate or sodium hydrogen phosphate.

**15.** A method according to any of claims 1 to 5 characterised in that said borate is of a metal of Group IA, Group IIA or Group IIB of the Periodic Table.

**16.** A method according to claim 15 characterised in that the borate is lithium borate, sodium borate, magnesium borate, calcium borate, barium borate or zinc borate.

**17.** A continuous method for preparing t-butyl alcohol according to any of the preceding claims wherein the isobutane is continuously reacted with molecular oxygen in a reaction zone under liquid phase oxidation reaction conditions to provide an initial reaction mixture comprising unreacted isobutane and isobutane oxidation reaction products, principally t-butyl hydroperoxide and t-butyl alcohol characterised in that it comprises:
(a) conducting said reaction in the presence of catalyst,
(b) continuously recovering t-butyl alcohol from said t-butyl alcohol fraction.

**18.** A method according to claim 17 comprising a solution of t-butyl hydroperoxide in t-butyl alcohol which contains from 5 to 30 wt.% of butyl hydroperoxide wherein unreacted isobutane is continuously separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that it comprises:
(a) 0.001 to 5 wt % based on the weight of the t-butyl hydroperoxide of a soluble rhenium catalyst compound and 1 to 3 parts by weight, based on the weight of the rhenium compound, of a bidentate ligand selected from 2,2′-dipyridyl and bis(salicylidene)ethylenediamine.
(b) conducting said hydroperoxide decomposition reaction in the presence of said thus-prepared hydroperoxide decomposition catalyst in said hydroperoxide decomposition zone in liquid phase with agitation under reaction conditions including a temperature within the range of 30° to 80°C. and autogeneous pressure, and
(c) continuously removing a stream of said hydroperoxide conversion product from said hydroperoxide conversion zone.
(d) said bidentate ligand having the formula as claimed in claim 2.

**19.** A continuous method according to claim 18 characterised in that
(a) the oxidation reaction conditions include a temperature within the range of 135°C to 155°C, a pressure of 300 to 800 psig. (2070 to 5520 kPa g.p.) and a holding time of 2 to 6 hours to provide an initial reaction mixture comprising unreacted isobutane and isobutane oxidation reaction products, principally t-butyl hydroperoxide and t-butyl alcohol,
(b) continuously separating unreacted isobutane from said initial reaction mixture to provide a charge stock,
(c) continuously feeding said charge stock to a hydroperoxide decomposition zone, adding said decomposition catalyst, and
(d) continuously conducting a catalytic hydroperoxide decomposition reaction in said hydroperoxide decomposition zone in liquid phase with agitation under reaction conditions at a temperature within

the range of 30° to 80°C. and autogenous pressure, to convert said t-butyl hydroperoxide to decomposition products, principally t-butyl alcohol, to provide a hydroperoxide conversion product.

**20.** A method according to claims 18 or 19 characterised in that the rhenium catalyst compound is rhenium heptoxide-p-dioxane.

**21.** A method according to claim 17 wherein unreacted isobutane is continuously separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that said hydroperoxide decomposition reaction is conducted:

(a) in the presence of 0.001 to 5 wt.%, based on the weight of said charge stock of an iron (III) or manganese (III) porphine catalyst, by contacting said t-butyl hydroperoxide in said hydroperoxide decomposition zone in liquid phase with agitation with said hydroperoxide decomposition catalyst in solution in said t-butyl alcohol under reaction conditions including a temperature within the range of 20° to 125°C. and autogeneous pressure,

(b) continuously removing a stream of said hydroperoxide conversion product from said hydroperoxide conversion zone; and

(c) continuously separating said hydroperoxide conversion product stream into an isobutane fraction and a t-butyl alcohol fraction.

**22.** A method according to claim 21 characterised in that the decomposition reaction is conducted in the additional presence of from 0.2 to 5 parts by weight, per part of said porphine catalyst of a $C_1$-$C_{18}$-alkyl thiol.

**23.** A method according to claim 21 characterised in that the reaction is conducted in the additional presence of from 0.2 to 5 parts by weight per part of said porphine catalyst of a heterocyclic amine.

**24.** A method according to any of claims 21 to 23 characterised in that said hydroperoxide decomposition reaction is conducted in the presence of 0.001 to 5 wt.%, based on the weight of said charge stock of a meso-tetra-phenylporphine carboxylate or halide, in the additional presence of from 0.2. to 5 parts by weight, per part of said porphine catalyst of a $C_1$-$C_{18}$ alkyl thiol and in the further presence of from 0.2 to 5 parts by weight, per part of said porphine catalyst of an imidazole or a $C_1$-$C_4$ 1-alkyl or 2-alkyl imidazole by contacting said t-butyl hydroperoxide in said hydroperoxide decomposition zone in liquid phase with agitation with said decomposition catalyst in solution in said t-butyl alcohol, under reaction conditions including a temperature within the range of 20° to 125°C. and autogeneous pressure.

**25.** A method according to claim 17 wherein the solution of t-butyl hydroperoxide and t-butyl alcohol contains from 10 to 40 wt.% of t-butyl hydroperoxide, wherein unreacted isobutane is continuously separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that it comprises:

(a) adding from 0.001 to 5 wt.%, based on the weight of the t-butyl hydroperoxide of chloroferric phthalocyanine or cobalt phthalocyanine and 0.2 to 1 parts by weight, based on the weight of the phthalocyanine of a rhenium compound selected from rhenium heptoxide-p-dioxane, ammonium rhenium tetroxide or rhenium decacarbonyl to said charge stock as said hydroperoxide decomposition catalyst,

(b) conducting said hydroperoxide decomposition reaction in the presence of said thus-prepared hydroperoxide decomposition catalyst in said hydroperoxide decomposition zone in liquid phase with agitation under reaction conditions including a temperature within the range of 20° to 40°C, and autogeneous pressure, and

(c) continuously removing a stream of said hydroperoxide conversion product from said hydroperoxide conversion zone.

**26.** A method according to claim 17, wherein said t-butyl hydroperoxide is continuously brought into contact with a catalytically effective amount of a phthalocyanine catalyst in a reaction zone under liquid phase reaction conditions including a temperature within the range of 20° to 120°C and a pressure of 0 to 1000 psig (6900 kPa g.p) characterised in that it comprises:
(a) using, as said reduction catalyst, 0.001 to 5 wt.%, based on the weight of the charge stock, of a metal phthalocyanine catalyst, the metal of said metal phthalocyanine catalyst being a heavy metal selected from Group IB, Group VIIB and Group VIIIB of the Periodic Table, and
(b) promoting the catalytic activity of said metal phthalocyanine catalyst with from 0.2 to 5 parts by weight, per part of said metal phthalocyanine catalyst of imidazole and
(c) continuously removing a stream of said conversion product from said reaction zone.

**27.** A method according to claim 26 characterised in that the stream is continuously separated into an isobutane fraction and a t-butyl alcohol fraction, the t-butyl alcohol is removed from said t-butyl alcohol fraction, and said isobutane fraction is continuously recycled to said reaction zone.

**28.** A method according to claim 17 containing a solution of t-butyl hydroperoxide in t-butyl alcohol with 2 to 90 wt.%. of t-butyl hydroperoxide wherein unreacted isobutane is continuously separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that it comprises:
(a) adding from 0.001 to 5 wt.%, based on the weight of the t-butyl hydroperoxide of chloroferric phthalocyanine, 1 to 1.5 parts by weight, per part of the chloroferric phthalocyanine of 2,3-dihydroxynaphthalene and 1 to 20 parts by weight, per part of 2,3-dihydroxynaphthalene of a $C_1$-$C_{18}$ alkyl thiol to said charge stock as said hydroperoxide decomposition catalyst,
(b) conducting said hydroperoxide decomposition reaction in the presence of said thus-prepared hydroperoxide decomposition catalyst in said hydroperoxide decomposition zone in liquid phase with agitation under reaction conditions including a temperature within the range of 20° to 125°C. and autogenous pressure, and
(c) continuously removing a stream of said hydroperoxide conversion product from said hydroperoxide conversion zone.

**29.** A method according to claim 17 comprising a solution of t-butyl hydroperoxide in t-butyl alcohol which contains from 5 to 30 wt.% of t-butyl hydroperoxide wherein unreacted isobutane is continuously separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that it comprises:
(a) adding to said charge stock as said hydroperoxide decomposition catalyst from 0.001 to 5 wt.%, based on the weight of the t-butyl hydroperoxide of chloroferric phthalocyanine and 1 to 5 parts by weight, based on the weight of the chloroferric phthalocyanine of an alkali metal base of an organic or inorganic acid having a pH greater than 7.5 when 0.10 mole is dissolved in 1 litre of water.
(b) conducting said hydroperoxide decomposition reaction in the presence of said thus-prepared hydroperoxide decomposition catalyst in said hydroperoxide decomposition zone in liquid phase with agitation under reaction conditions including a temperature within the range of 20° to 125°C. and autogenous pressure.
(c) continuously removing a stream of said hydroperoxide conversion product from such hydroperoxide conversion zone.

**30.** A method according to claim 29 characterised in that said chloroferric phthalocyanine derives from lithium, sodium or potassium acetate, carbonate, bicarbonate or hydrogen phosphate.

**31.** A method according to claim 17 comprising a solution of t-butyl hydroperoxide in t-butyl alcohol which contains from 2 to 90 wt% of t-butyl hydroperoxide wherein unreacted isobutane is continuously

separated from said initial reaction mixture to provide a charge stock comprising a solution of said t-butyl hydroperoxide in said t-butyl alcohol, wherein said charge stock is continuously charged to a hydroperoxide decomposition zone, and wherein a catalytic hydroperoxide decomposition reaction is continuously conducted in said decomposition reaction zone to convert said t-butyl hydroperoxide to decomposition products, principally t-butyl alcohol, to provide a hydroperoxide conversion product, characterised in that it comprises:

(a) adding from 0.001 to 5 wt.%, based on the weight of the t-butyl hydroperoxide of chloroferric phthalocyanine and about 1 to 6 parts by weight per part of the chloroferric phthalocyanine of a borate of a metal of Group IA, Group IIA or Group IIB of the Periodic Table to said charge stock as said hydroperoxide decomposition catalyst.

(b) conducting said hydroperoxide decomposition reaction in the presence of said thus-prepared hydroperoxide decomposition catalyst in said hydroperoxide decomposition zone in liquid phase with agitation under reaction conditions including a temperature within the range of 20° to 125°C. and autogenous pressure.

(c) continuously removing a stream of said hydroperoxide conversion product from said hydroperoxide conversion zone.

**32.** A method according to claim 30 comprising a solution of t-butyl hydroperoxide in t-butyl alcohol which contains from 20 to 50 wt.% of t-butyl hydroperoxide characterised in that it comprises adding

(a) 1 to 6 parts by weight, per part of the chloroferric phthalocyanine of a lithium, sodium, magnesium, zinc, or calcium borate to said charge stock as said hydroperoxide decomposition catalyst.

**33.** A method according to claim 26 characterised in that the metal phthalocyanine is manganous phthalocyanine or chloroferric phthalocyanine.

## Revendications

**1.** Procédé ce conversion en phase liquide de l'hydroperoxyde de t-butyle en produits de décomposition, en particulier l'alcool t-butylique, en mettant en contact une charge de départ à base d'hydroperoxyde de t-butyle avec une quantité à effet catalytique d'un catalyseur dans une zone réactionnelle en agitant, caractérisé en ce que le catalyseur est choisi parmi :

(a) un composé dérivé de métal et de porphine,

(b) un composé catalytique soluble dérivé du ruthénium, activé avec un ligand bidenté, et

(c) un composé dérivé de métal et de phtalocyanine activé.

**2.** Procédé selon la revendication 1, caractérisé en ce que le ligand bidenté, correspond à la formule suivante :

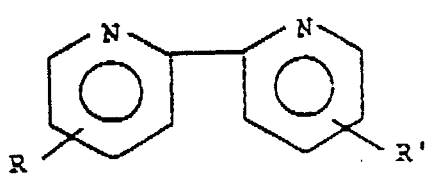

ou

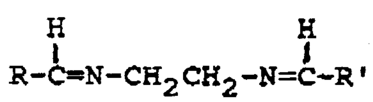

dans lesquelles R et R' peuvent être identiques ou différents, et ils sont choisis parmi un atome d'hydrogène, et les groupes méthyle, éthyle, propyle, phényle et chlorophényle.

**3.** Procédé selon la revendication 1, caractérisé en ce que le catalyseur dérivé de métal et de phtalocyanine ou le composé dérivé de métal et de porphine, est activé avec une amine et/ou un mercaptan.

**4.** Procédé selon la revendication 1, caractérisé en ce que la catalyseur dérivé de métal et de phtalocyanine est activé avec un inhibiteur de radicaux libres et/ou un mercaptan, avec une base ou un borate, le métal étant choisi parmi les groupes IB, VIIB ou VIIIB du tableau périodique.

**5.** Procédé selon la revendication 4, caractérisé en ce que la charge t-tubylique de départ, consiste en

une solution dans l'alcool t-butylique.

6. Procédé selon la revendication 5, caractérisé en ce que la solution à base d'alcool t-butylique consiste en un catalyseur soluble dérivé du ruthénium et en un ligand bidenté consistant en le 2,2'-dipyridyle ou la bis(salicylidéne)éthylènediamine.

7. Procédé selon la revendication 6, caractérisé en ce que le composé catalytique soluble dérivé du ruthénium, est l'acétylacétonate de rythénium.

8. Procédé selon l'une quelconque des revendications 1 à 5, caractérisé en ce que le mercaptan est un thiol à groupe alkyle en $C_1$-$C_{18}$.

9. Procédé selon l'une quelconque des revendications 1 à 5, caractérisé en ce que l'amine est la pyridine, la quinoléine, l'isoquinoléine, l'imidazole, ou un 1-alkyl ou 2-alkyl imidazole, le groupe alkyle contenant de 1 à 4 atomes de carbone.

10. Procédé selon la revendication 5, caractérisé en ce que la solution à base d'alcool t-butylique comprend un catalyseur à base de phtalocyanine chloroferrique, manganeuse ou dérivée du cobalt, et un composé soluble dérivé du rhénium, choisi parmi l'heptylate de rhénium-p-dioxanne, le tétraoxyde de rhénium et d'ammonium ou le rhénium décacarbonyle.

11. Procédé selon l'une quelconque des revendications 1 à 5, caractérisé en ce que la base est au moins partiellement soluble dans l'alcool t-butylique.

12. Procédé selon la revendication 11, caractérisé en ce que la base est une base inorganique ayant un pH supérieur à 7,5 selon une concentration de 0,10 mole dissoute dans 1 litre d'eau.

13. Procédé selon la revendication 11 ou 12, caractérisé en ce que la base est un carbonate, un bicarbonate, un acétate ou un hydrogénophosphate de métal alcalin.

14. Procédé selon la revendication 13, caractérisé en ce que la base est l'acétate de sodium, l'acétate de potassium, le bicarbonate de sodium, le bicarbonate de lithium, le carbonate de lithium ou l'hydrogénophosphate de sodium.

15. Procédé selon l'une quelconque des revendications 1 à 5, caractérisé en ce que le borate est dérivé d'un métal du groupe IA, IIA ou IIB du tableau périodique.

16. Procédé selon la revendication 15, caractérisé en ce que le borate est le borate de lithium, le borate de sodium, le borate de magnésium, le borate de calcium, le borate de baryum ou le borate de zinc.

17. Procédé continu de préparation d'alcool t-butylique selon l'une quelconque des revendications précédentes, dans lequel on fait réagir de manière continue de l'isobutane avec de l'oxygène moléculaire dans une zone réactionnelle sous des conditions réactionnelles d'oxydation en phase liquide, pour procurer un mélange réactionnel initial comprenant de l'isobutane n'ayant pas réagi et des produits de réaction d'oxydation de l'isobutane, principalement de l'hydroperoxyde de t-butyle et de l'alcool t-butylique, caractérisé en ce qu'il comprend les étapes consistant :
(a) à affectuer cette réaction en présence d'un catalyseur,
(b) à récupérer de manière continue l'alcool t-butylique à partir de la fraction à base d'alcool t-butylique.

18. Procédé selon la revendication 17, comprenant une solution d'hydroperoxyde de t-butyle dans de l'alcool t-butylique qui contient de 3 à 30 % en poids d'hydroperoxyde de butyle, l'isobutane n'ayant pas réagi étant séparé de manière continue de ce mélange réactionnel initial, afin de former une charge de départ comprenant une solution d'hydroperoxyde de t-butyle dans cet alcool t-butylique, la charge de départ étant introduite de manière continue dans une zone de décomposition d'hydroperoxyde, et une réaction de décomposition catalytique de l'hydroperoxyde étant effectuée de manière continue dans cette zone de réaction de décomposition, pour convertir l'hydroperoxyde de t-butyle en produits de décomposition, principalement en alcool t-butylique, afin d'obtenir un produit de conversion

d'hydroperoxyde, caractérisé en ce qu'il comprend les étapes consistant :

(a) à ajouter de 0,001 à 5 % en poids par rapport au poids de l'hydroperoxyde de t-butyle, d'un composé catalytique soluble dérivé du rhénium, et de 1 à 3 parties en poids, par rapport au poids du composé dérivé du rhénium, d'un ligand bidenté choisi parmi le 2,2'-dipyridyle et la bis-(salicylidène)éthylènediamine,

(b) à effectuer cette réaction de décomposition d'hydroperoxyde, en présence du catalyseur de décomposition d'hydroperoxyde ainsi préparé, dans la zone de décomposition d'hydroperoxyde, en phase liquide et en agitant dans des conditions réactionnelles comprenant une température de 30 à 80 ° C et sous pression autogène, et

(c) à éliminer de manière continue un courant de ce produit de conversion d'hydroperoxyde à partir de la zone de conversion d'hydroperoxyde,

(d) le ligand bidenté correspondant à la formule mentionnée dans la revendication 2.

**19.** Procédé continu selon la revendication 18, caractérisé en ce que :

(a) les conditions de réaction d'oxydation comprennent une température de 135 ° C à 155 ° C, une pression de 300 à 800 psig (pression relative : 2 070 à 5 520 kPa), et un temps de maintien de 2 à 6 h, pour obtenir un mélange réactionnel initial comprenant de l'isobutane n'ayant pas réagi et des produits de réaction d'oxydation de l'isobutane, principalement de l'hydroperoxyde de t-butyle et de l'alcool t-butylique,

(b) on sépare de manière continue l'isobutane n'ayant pas réagi du mélange réactionnel initial, afin de former une charge de départ,

(c) on introduit de manière continue cette charge de départ, dans une zone de décomposition hydroperoxyde, on ajoute le catalyseur de décomposition, et

(d) on effectue de manière continue une réaction de décomposition catalytique d'hydroperoxyde dans la zone de décomposition d'hydroperoxyde, en phase liquide et en agitant dans des conditions réactionnelles comprenant une température de 30° à 80 ° C et sous pression autogène, pour convertir l'hydroperoxyde de t-butyle en produits de décomposition, principalement l'alcool t-butylique, afin d'obtenir un produit de conversion d'hydroperoxyde.

**20.** Procédé selon la revendication 18 ou 19, caractérisé en ce que le composé catalytique dérivé du rhénium, est l'heptylate de rhénium-p-dioxanne.

**21.** Procédé selon la revendication 17, dans lequel l'isobutane n'ayant pas réagi est séparé de manière continue du mélange réactionnel initial, afin de former une charge de départ comprenant une solution de cet hydroperoxyde de t-butyle dans l'alcool t-butylique, la charge de départ étant introduite de manière continue dans une zone de décomposition d'hydroperoxyde, et une réaction catalytique de décomposition d'hydroperoxyde est effectuée de manière continue dans la zone de réaction de décomposition, afin de convertir l'hydroperoxyde de t-butyle en produits de décomposition, principalement l'alcool t-butylique, et obtenir un produit de conversion d'hydroperoxyde, caractérisé en ce que la réaction de décomposition d'hydroperoxyde, est effectuée :

(a) en présence de 0,001 à 5 % en poids, par rapport au poids de la charge de départ, d'un catalyseur dérivé de porphine et du fer (III) ou du manganèse (III), en mettant en contact l'hydroperoxyde de t-butyle dans la zone de décomposition d'hydroperoxyde, en phase liquide et en agitant, avec le catalyseur de décomposition d'hydroperoxyde, en solution dans l'alcool t-butylique, dans des conditions réactionnelles comprenant une température de 20° à 125 ° C et sous pression autogène,

(b) en éliminant de manière continue un courant de ce produit de conversion d'hydroperoxyde à partir de la zone de conversion d'hydroperoxyde ; et

(c) en séparant de manière continue le courant de produit de conversion d'hydroperoxyde, en une fraction à base d'isobutane et une fraction à base d'alcool t-butylique.

**22.** Procédé selon la revendication 21, caractérisé en ce que la réaction de décomposition est effectuée en la présence supplémentaire de 0,2 à 5 parties en poids, par partie de ce catalyseur dérivé de phosphine, d'un thiol à groupe alkyle en $C_1$-$C_{18}$.

**23.** Procédé selon la revendication 21, caractérisé en ce que la réaction est effectuée en la présence supplémentaire de 0,2 à 5 parties en poids par partie du catalyseur dérivé de phosphine, d'une amine hétérocyclique.

**24.** Procédé selon l'une quelconque des revendications 21 à 23, caractérisé en ce que la réaction de décomposition d'hydroperoxyde, est effectuée en présence de 0,001 à 5 % en poids par rapport au poids de la charge de départ, d'un carboxylate ou d'un halogénure de méso-tétraphénylporphine, en la présence supplémentaire de 0,2 à 5 parties en poids par partie du catalyseur dérivé de phosphine, d'un thiol à groupe alkyle en $C_1$-$C_{18}$, et en outre en présence de 0,2 à 5 parties en poids par partie du catalyseur dérivé de phosphine, d'un imidazole ou d'un 1-alkyl ou 2-alkyl en $C_1$-$C_4$-imidazole, en mettant en contact cet hydroperoxyde de t-butyle dans la zone de décomposition d'hydroperoxyde, en phase liquide et en agitant, avec le catalyseur de décomposition en solution dans l'alcool t-butylique, dans des conditions réactionnelles comprenant une température de 20° à 125 °C et sous pression autogène.

**25.** Procédé selon la revendication 17, dans lequel la solution d'hydroperoxyde de t-butyle et d'alcool t-butylique, contient de 10 à 40 % en poids d'hydroperoxyde de t-butyle, l'isobutane n'ayant pas réagi et étant séparé de manière continue du mélange réactionnel initial, pour former une charge de départ comprenant une solution de l'hydroperoxyde de t-butyle dans l'alcool t-butylique, la charge de départ étant introduite de manière continue dans une zone de décomposition d'hydroperoxyde, et une réaction catalytique de décomposition d'hydroperoxyde étant effectuée de manière continue dans la zone de réaction de décomposition, pour convertir l'hydroperoxyde t-butyle en produits de réaction, principalement en alcool t-butylique, et procurer un produit de conversion d'hydroperoxyde, caractérisé en ce qu'il comprend les étapes consistant :

(a) à ajouter de 0,001 à 5 % en poids par rapport au poids de l'hydroperoxyde de t-butyle, de phtalocyanine chloroferrique ou de cobalt phtalocyanine, et de 0,2 à 1 partie en poids par rapport au poids de la phtalocyanine, d'un composé dérivé du rhénium choisi parmi l'heptylate de rhénium-p-dioxanne, le tétraoxyde de rhénium et d'ammonium ou le rhénium décacarbonyle dans la charge de départ en tant que le catalyseur de décomposition d'hydroperoxyde,

(b) à effectuer la réaction de décomposition d'hydroperoxyde en présence du catalyseur de décomposition d'hydroperoxyde ainsi préparé, dans la zone de décomposition d'hydroperoxyde en phase liquide et en agitant dans des conditions réactionnelles comprenant une température de 20° à 40 °C et sous pression autogène, et

(c) à éliminer de manière continue un courant de ce produit de conversion d'hydroperoxyde à partir de la zone de conversion d'hydroperoxyde.

**26.** Procédé selon la revendication 17, dans lequel l'hydroperoxyde de t-butyle est mis de manière continue en contact avec une quantité à effet catalytique d'un catalyseur dérivé de phtalocyanine dans une zone réactionnelle sous des conditions réactionnelles en phase liquide comprenant une température de 20° à 120° C et une pression de 0 à 1 000 psig (pression relative : 6.900 kpa), caractérisé en ce qu'il comprend les étapes consistant :

(a) à employer comme catalyseur de réduction, de 0,001 à 5 % en poids par rapport au poids de la charge de départ, un catalyseur dérivé de phtalocyanine et de métal, le métal de ce catalyseur dérivé de métal et de phtalocyanine étant un métal lourd choisi parmi les groupes IB, VIIB et VIIIB du tableau périodique, et

(b) à accroître l'activité catalytique du catalyseur dérivé de métal et de phtalocyanine, avec de 0,2 à 5 parties en poids par partie du catalyseur dérivé de métal et de phtalocyanine, d'un imidazole, et

(c) à éliminer de manière continue un courant de ce produit de conversion, à partir de la zone réactionnelle.

**27.** Procédé selon la revendication 26, caractérisé en ce que le courant est séparé de manière continue en une fraction à base d'isobutane et une fraction à base d'alcool t-butylique, l'alcool t-butylique étant séparé de la fraction à base d'alcool t-butylique, et la fraction à base d'isobutane étant recyclée de manière continue dans la zone réactionnelle.

**28.** Procédé selon la revendication 17, dans lequel on met en oeuvre une solution d'hydroperoxyde de t-butyle dans l'alcool t-butylique contenant de 2 à 90 % en poids d'hydroperoxyde de t-butyle, l'isobutane n'ayant pas réagi étant séparé de manière continue de ce mélange réactionnel initial, pour former une charge de départ comprenant une solution de l'hydroperoxyde de t-butyle dans l'alcool t-butylique, la charge de départ étant introduite de manière continue dans une zone de décomposition d'hydroperoxyde, et une réaction catalytique de décomposition d'hydroperoxyde étant effectuée de manière continue dans la zone de réaction de décomposition, afin de convertir l'hydroperoxyde de t-butyle en produits de décomposition, principalement en alcool t-butylique, et procurer un produit de conversion d'hydroperoxyde, caractérisé en ce qu'il comprend les étapes consistant :

(a) à ajouter de 0,001 à 5 % en poids par rapport au poids de l'hydroperoxyde de t-butyle, de phtalocyanine chloroferrique, de 1 à 1,5 parties en poids par partie de la phtalocyanine chloroferrique, de 2,3-dihydroxynaphtalène, et 1 à 20 parties en poids par partie de 2,3-dihydroxynaphtaléne, d'un thiol à groupe alkyle en $C_1$-$C_{18}$, dans la charge de départ en tant que catalyseur de décomposition d'hydroperoxyde,

(b) à effectuer cette réaction de décomposition d'hydroperoxyde en présence du catalyseur de décomposition d'hydroperoxyde ainsi préparé dans la zone de décomposition d'hydroperoxyde, en phase liquide et en agitant dans des conditions réactionnelles comprenant une température de 20° à 125 °C et sous pression autogène, et

(c) à éliminer de manière continue un courant de ce produit de conversion d'hydroperoxyde, à partir de la zone de conversion d'hydroperoxyde.

**29.** Procédé selon la revendication 17, dans lequel on met en oeuvre une solution d'hydroperoxyde de t-butyle dans de l'alcool t-butylique contenant de 5 à 30 % en poids d'hydroperoxyde de t-butyle, l'isobutane n'ayant pas réagi étant séparé de manière continue du mélange réactionnel initial, pour former une charge de départ comprenant une solution de l'hydroperoxyde de t-butyle dans l'alcool t-butylique, la charge de départ étant introduite de manière continue dans une zone de décomposition d'hydroperoxyde, et une réaction catalytique de décomposition d'hydroperoxyde étant effectuée de manière continue dans la zone de réaction de décomposition, afin de convertir l'hydroperoxyde de t-butyle en produits de décomposition, principalement en alcool t-butylique, et procurer un produit de conversion d'hydroperoxyde, caractérisé en ce qu'il comprend les étapes consistant :

(a) à ajouter dans cette charge de départ en tant que catalyseur de décomposition d'hydroperoxyde, de 0,001 à 5 % en poids par rapport au poids de l'hydroperoxyde de t-butyle, de phtalocyanine (chloroferrique, et de 1 à 5 parties en poids par rapport au poids de la phtalocyanine chloroferrique, d'une base dérivée d'un métal alcalin et d'un acide organique ou inorganique, ayant un pH supérieur à 7,5 pour une concentration de 0,10 mole dissoute dans 1 litre d'eau,

(b) à effectuer cette réaction de décomposition d'hydroperoxyde, en présence du catalyseur de décomposition d'hydroperoxyde ainsi préparé dans la zone de décomposition d'hydroperoxyde, en phase liquide et en agitant dans des conditions réactionnelles comprenant une température de 20° à 125 °C et sous pression autogène, et

(c) à éliminer de manière continue un courant de ce produit de conversion d'hydroperoxyde à partir de cette zone de conversion d'hydroperoxyde.

**30.** Procédé selon la revendication 29, caractérisé en ce que la phtalocyanine chloroferrique est activée avec de l'acétate, du carbonate, du bicarbonate ou de l'hydrogénophosphate de lithium, de sodium ou de potassium.

**31.** Procédé selon la revendication 17, dans lequel on met en oeuvre une solution d'hydroperoxyde de t-butyle dans de l'alcool t-butylique qui contient de 2 à 90 % en poids d'hydroperoxyde de t-butyle, l'isobutane n'ayant pas réagi étant séparé de manière continue de ce mélange réactionnel initial pour former une charge de départ comprenant une solution de l'hydroperoxyde de t-butyle dans l'alcool butylique, la charge de départ étant introduite de manière continue dans une zone de décomposition d'hydroperoxyde, et une réaction catalytique de décomposition d'hydroperoxyde étant effectuée de manière continue dans la zone de réaction de décomposition, afin de convertir l'hydroperoxyde de t-butyle en produits de décomposition, principalement en alcool t-butylique, et procurer un produit de conversion d'hydroperoxyde, caractérisé en ce qu'il comprend les étapes consistant :

(a) à ajouter de 0,001 à 5 % en poids par rapport au poids de l'hydroperoxyde de t-butyle, de phtalocyanine chloroferrique, et d'environ 1 à 6 parties en poids par partie de la phtalocyanine chloroferrique, d'un borate d'un métal du groupe IA, du groupe IIA ou du groupe IIB du tableau périodique, dans la charge de départ en tant que catalyseur de décomposition d'hydroperoxyde,

(b) à effectuer la réaction de décomposition d'hydroperoxyde en présence du catalyseur de décomposition d'hydroperoxyde ainsi préparé, dans la zone de décomposition d'hydroperoxyde, en phase liquide et en agitant dans des conditions réactionnelles comprenant une température de 20° à 125 °C et sous pression autogène, et

(c) à éliminer de manière continue un courant du produit de conversion d'hydroperoxyde, à partir de la zone de conversion d'hydroperoxyde.

32. Procédé selon la revendication 30, dans lequel on met en oeuvre une solution d'hydroperoxyde de t-butyle dans l'alcool t-butylique, contenant de 20 à 50 % en poids d'hydroperoxyde de t-butyle, caractérisé en ce qu'il comprend l'étape consistant :
(a) à ajouter de 1 à 6 parties en poids par partie de la phtalocyanine chloroferrique, de borate de lithium, de sodium, de magnésium, de zinc ou de calcium, dans la charge de départ en tant que catalyseur de décomposition d'hydroperoxyde.

33. Procédé selon la revendication 26, caractérisé en ce que la phtalocyanine dérivée de métal, est la phtalocyanine manganeuse ou la phtalocyanine cyanoferrique.

**Patentansprüche**

1. Verfahren zur Umwandlung von t-Butylhydroperoxid zu Zersetzungsprodukten, hauptsächlich t-Butylalkohol, indem ein t-Butylhydroperoxid-Einsatzmaterial in flüssiger Phase unter Rühren in einer Reaktionszone mit einer katalytisch wirksamen Menge eines Katalysators in Kontakt gebracht wird, dadurch gekennzeichnet, daß der Katalysator ausgewählt ist aus:
(a) einer Metall-Porphin-Verbindung
(b) einer löslichen Ruthenium-Katalysator-Verbindung, aktiviert mit einem zweizähnigen Liganden, und
(c) einer aktivierten Metall-Phthalocyanin-Verbindung.

2. Verfahren nach Anspruch 1, dadurch gekennzeichnet, daß der zweizähnige Ligand die Formel besitzt:

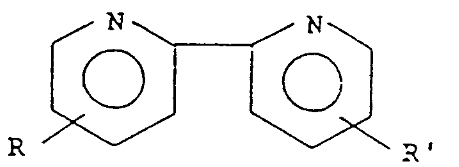

oder

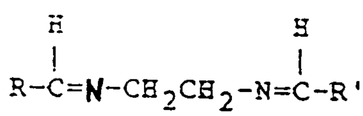

in der R und R' identisch oder verschieden sein können und ausgewählt sind aus Wasserstoff, Methyl, Ethyl, Propyl, Phenyl und Chlorphenyl.

3. Verfahren nach Anspruch 1, dadurch gekennzeichnet, daß besagter Metall-Phthalocyanin-Katalysator oder eine Metall-Porphin-Verbindung mit einem Amin oder/und einem Mercaptan aktiviert wird.

4. Verfahren nach Anspruch 1, dadurch gekennzeichnet, daß besagter Metall-Phthalocyanin-Katalysator mit einem Inhibitor für freie Radikale und/oder einem Mercaptan, mit einer Base oder mit Borat aktiviert wird, wobei besagtes Metall ausgewählt ist aus den Gruppen IB, VIIB oder VIIIB der Tabelle des Periodensystems.

5. Verfahren nach Anspruch 4, dadurch gekennzeichnet, daß das t-Butyl-Einsatzmaterial in Lösung mit t-Butylalkohol vorliegt.

6. Verfahren nach Anspruch 5, dadurch gekennzeichnet, daß die t-Butylalkohollösung aus einem löslichen Rutheniumkatalysator und einem zweizähnigen 2,2'-Dipyridyl- oder Bis(salicyliden)ethylendiamin-Liganden besteht.

7. Verfahren nach Anspruch 6, dadurch gekennzeichnet, daß besagte Ruthenium-Katalysatorverbindung Rutheniumacetylacetonat ist.

8. Verfahren nach einem der Ansprüche 1 oder 5, dadurch gekennzeichnet, daß besagtes Mercaptan ein $C_1$-$C_{18}$-Alkylthiol ist.

9. Verfahren nach einem der Ansprüche 1 bis 5, dadurch gekennzeichnet, daß besagtes Amin Pyridin, Chinolin, Isochinolin, Imidazol oder 1-Alkyl- oder 2-Alkylimidazol ist, wobei die Alkylgruppe von 1 bis 4 Kohlenstoffatome enthält.

**10.** Verfahren nach Anspruch 5, dadurch gekennzeichnet, daß die t-Butylalkohollösung aus einem Chloroferri-, einem Mangan- oder einem Kobalt-Phtalocyanin-Katalysator und einer löslichen Rheniumverbindung besteht, die ausgewählt ist aus Rheniumheptoxid-p-Dioxan, Ammoniumrheniumtetroxid oder Rheniumdecacarbonyl.

**11.** Verfahren nach einem der Ansprüche 1 bis 5, dadurch gekennzeichnet, daß die besagte Base wenigstens in besagtem t-Butylalkohol löslich ist.

**12.** Verfahren nach Anspruch 11, dadurch gekennzeichnet, daß besagte Base eine anorganische Base mit einem pH von mehr als 7,5 ist, wenn 0,10 Mol in 1 Liter Wasser gelöst sind.

**13.** Verfahren nach Anspruch 11 oder 12, dadurch gekennzeichnet, daß besagte Base ein Alkalimetallcarbonat, -bicarbonat, -acetat oder -hydrogenphosphat ist.

**14.** Verfahren nach Anspruch 13, dadurch gekennzeichnet, daß besagte Base Natriumacetat, Kaliumacetat, Kaliumbicarbonat, Lithiumbicarbonat oder Natriumhydrogenphosphat ist.

**15.** Verfahren nach einem der Ansprüche 1 bis 5, dadurch gekennzeichnet, daß besagtes Borat von einem Metall der Gruppe IA, Gruppe IIA oder Gruppe IIB der Tabelle des Periodensystems ist.

**16.** Verfahren nach Anspruch 15, dadurch gekennzeichnet, daß das Borat Lithiumborat, Natriumborat, Magnesiumborat, Calciumborat, Bariumborat oder Zinkborat ist.

**17.** Kontinuierliches Verfahren zur Herstellung von t-Butylalkohol nach einem der vorangehenden Ansprüche, wobei das Isobutan kontinuierlich in einer Reaktionszone unter Flüssigphasen-Oxidationsreaktionsbedingungen mit molekularem Sauerstoff umgesetzt wird, um eine anfängliche Reaktionsmischung zur Verfügung zu stellen, die nicht-umgesetztes Isobutan und Reaktionsprodukte der Oxidation von Isobutan umfaßt, hauptsächlich t-Butylhydroperoxid und t-Butylalkohol, dadurch gekennzeichnet, daß es folgendes umfaßt:
a) Durchführen besagter Reaktion in Gegenwart eines Katalysators,
b) kontinuierliches Gewinnen von t-Butylalkohol aus besagter t-Butylalkohol-Fraktion.

**18.** Verfahren nach Anspruch 17, umfassend eine Lösung aus t-Butylhydroperoxid in t-Butylalkohol, die von 5 bis 30 Gew.-% Butylhydroperoxid enthält, wobei nicht-umgesetztes Isobutan kontinuierlich von besagter anfänglicher Reaktionsmischung abgetrennt wird, um ein Einsatzmaterial zur Verfügung zu stellen, das eine Lösung von besagtem t-Butylhydroperoxid in besagtem t-Butylalkohol umfaßt, wobei besagtes Einsatzmaterial kontinuierlich einer Hydroperoxid-Zersetzungszone zugeführt wird und wobei eine katalytische Hydroperoxid-Zersetzungsreaktion in besagter Zersetzungsreaktionszone kontinuierlich durchgeführt wird, um besagtes t-Butylhydroperoxid zu Zersetzungsprodukten, hauptsächlich t-Butylalkohol umzuwandeln, um ein Hydroperoxid-Umwandlungsprodukt zur Verfügung zu stellen, dadurch gekennzeichnet, daß es folgendes umfaßt:
(a) 0,001 bis 5 Gew.-%, bezogen auf das Gewicht des t-Butylhydroperoxids, einer löslichen Rhenium-Katalysatorverbindung und 1 bis 3 Gewichtsteile, bezogen auf das Gewicht der Rheniumverbindung, eines zweizähnigen Liganden, ausgewählt aus 2,2'-Dipyridyl und Bis(salicyliden)-ethylendiamin.
(b) Durchführen besagter Hydroperoxid-Zersetzungsreaktion in Gegenwart von besagtem so hergestellten Hydroperoxid-Zersetzungskatalysator in besagter Hydroperoxid-Zersetzungszone in flüssiger Phase unter Rühren unter Reaktionsbedingungen, die eine Temperatur im Bereich von 30° bis 80° C und autogenen Druck einschließen, und
(c) kontinuierliches Entfernen eines Stroms von besagtem Hydroperoxid-Umwandlungsprodukt aus besagter Hydroperoxid-Umwandlungszone.
(d) wobei besagter zweizähniger Ligand die Formel besitzt, wie sie in Anspruch 2 beansprucht ist.

**19.** Kontinuierliches Verfahren nach Anspruch 18, dadurch gekennzeichnet, daß
(a) die Oxidationsreaktionsbedingungen eine Temperatur im Bereich von 135° C bis 155° C, einen Druck von 300 bis 800 psig (2010 bis 5520 kPa g.p.) und eine Haltezeit von 2 bis 6 Stunden einschließen, um eine anfängliche Reaktionsmischung zur Verfügung zu stellen, die nicht-umgesetztes Isobutan und Reaktionsprodukte aus der Oxidation von Isobutan, hauptsächlich t-Butylhydroperoxid und t-Butylalkohol, umfaßt.

(b) kontinuierliches Abtrennen nicht-umgesetzten Isobutans von besagter anfänglichen Reaktionsmischung, um ein Einsatzmaterial zur Verfügung zu stellen,

(c) kontinuierliches zuführen besagten Einsatzmaterials zu einer Hydroperoxid-Zersetzungszone, Zuführen besagten Zersetzungskatalysators, und

(d) kontinuierliches Durchführen eine katalytischen Hydroperoxid-Zersetzungsreaktion in besagter Hydroperoxid-Zersetzungszone in flüssiger Phase unter Rühren unter Reaktionsbedingungen bei einer Temperatur im Bereich von 30° bis 80°C und autogenem Druck, um besagtes t-Butylhydroperoxid zu Zersetzungsprodukten, hauptsächlich t-Butylalkohol, umzuwandeln, um ein Hydroperoxid-Umwandlungsproduktzur Verfügung zu stellen.

**20.** Verfahren nach den Ansprüchen 18 oder 19, dadurch gekennzeichnet, daß die Rhenium-Katalysatorverbindung Rheniumheptoxid-p-Dioxan ist.

**21.** Verfahren nach Anspruch 17, bei dem nicht-umgesetztes Isobutan kontinuierlich von besagter anfänglichen Reaktionsmischung abgetrennt wird, um ein Einsatzmaterial zur Verfügung zu stellen, das eine Lösung von besagtem t-Butylhydroperoxid in besagtem t-Butylalkohol umfaßt, wobei besagtes Einsatzmaterial kontinuierlich zu einer Hydroperoxid-Zersetzungszone zugeführt wird und wobei eine katalytische Hydroperoxid-Zersetzungsreaktion kontinuierlich in besagter Zersetzungsreaktionszone durchgeführt wird, um besagtes t-Butylhydroperoxid zu Zersetzungsprodukten, hauptsächlich t-Butyl-alkohol, umzuwandeln, um ein Hydroperoxid-Umwandlungsprodukt zur Verfügung zu stellen, dadurch gekennzeichnet, daß besagte Hydroperoxid-Zersetzungsreaktion folgendermaßen durchgeführt wird:

(a) in Gegenwart von 0,001 bis 5 Gew.-%, bezogen auf das Gewicht besagten Einsatzmaterials, eines Eisen (III)- oder Mangan (III)-Porphin-Katalysators, indem besagtes t-Butylhydroperoxid in besagter Hydroperoxid-Zersetzungszone in flüssiger Phase unter Rühren mit besagtem Hydroperoxid-Zersetzungskatalysator in Lösung mit besagtem t-Butylalkohol unter Reaktionsbedingungen in Kontakt gebracht wird, die eine Temperatur im Bereich von 200 bis 125°C und autogenen Druck einschließen,

(b) kontinuierliches Entfernen eines Stroms besagten Hydroperoxid-Umwandlungsproduktes aus besagter Hydroperoxid-Umwandlungszone; und

(c) kontinuierliches Auftrennen besagten Hydroperoxid-Umwandlungsproduktstromes in eine Isobutan-Fraktion und eine t-Butylalkohol-Fraktion.

**22.** Verfahren nach Anspruch 21, dadurch gekennzeichnet, daß die Zersetzungsreaktion zusätzlich in Gegenwart von 0,2 bis 5 Gewichtsteilen, pro Teil besagten Porphin-Katalysators, eines $C_1$-$C_{18}$-Alkylthiols durchgeführt wird.

**23.** Verfahren nach Anspruch 21, dadurch gekennzeichnet, daß die Reaktion zusätzlich in Gegenwart von 0,2 bis 5 Gewichtsteilen, pro Teil besagten Porphin-Katalysators, eines heterocyclischen Amins durchgeführt wird.

**24.** Verfahren nach einem der Ansprüche 21 bis 23, dadurch gekennzeichnet, daß besagte Hydroperoxid-Zersetzungsreaktion in Gegenwart von 0,001 bis 5 Gew.-%, bezogen auf das Gewicht besagten Einsatzmaterials, eines meso-Tetraphenylporphincarboxylats oder - halogenids, zusätzlich in Gegenwart von 0,2 bis 5 Gewichtsteilen, pro Teil besagten Porphin-Katalysators, eines $C_1$-$C_{18}$-Alkylthiols und weiterhin in Gegenwart von 0,2 bis 5 Gewichtsteilen, pro Teil besagten Porphin-Katalysators, eines Imidazols oder eines $C_1$-$C_4$-1-Alkyl- oder 2-Alkylimidazols durch In-Kontakt-Bringen besagten t-Butylhydroperoxids in besagter Hydroperoxid-Zersetzungszone in flüssiger Phase unter Rühren mit besagtem Zersetzungskatalysator in Lösung in besagtem t-Butylalkohol unter Reaktionsbedingungen durchgeführt wird, die eine Temperatur im Bereich von 20° bis 125°C und autogenen Druck einschließen.

**25.** Verfahren nach Anspruch 17, bei dem die Lösung von t-Butylhydroperoxid und t-Butylalkohol von 10 bis 40 Gew.-% t-Butylhydroperoxid enthält, nicht-umgesetztes Isobutan kontinuierlich von besagter anfänglichen Reaktionsmischung abgetrennt wird, um ein Einsatzmaterial zur Verfügung zu stellen, das eine Lösung von besagtem t-Butylhydroperoxid in besagtem t-Butylalkohol umfaßt, besagtes Einsatzmaterial kontinuierlich einer Hydroperoxidzersetzungszone zugeführt wird und eine katalytische Hydroperoxid-Zersetzungsreaktion kontinuierlich in besagter Zersetzungsreaktionszone durchgeführt wird, um besagtes t-Butylhydroperoxid zu Zersetzungsprodukten, hauptsächlich t-Butylalkohol, umzuwandeln, um ein Hydroperoxid-Umwandlungsprodukt zur Verfügung zu stellen, dadurch gekennzeichnet, daß es folgendes umfaßt:

(a) Zugabe von 0,001 bis 5 Gew.-%, bezogen auf das Gewicht des t-Butylhydroperoxids, Chloroferri-Phthalocyanin oder Kobalt-Phthalozyanin und 0,2 bis 1 Gewichtsteilen, bezogen auf das Gewicht des Phthalocyanins, einer Rheniumverbindung, ausgewählt aus Rheniumheptoxid-p-Dioxan, Ammoniumrheniumtetroxid oder Rheniumdecacarbonyl, zu besagtem Einsatzmaterial als besagter Hydroperoxid-Zersetzungskatalysator,

(b) Durchführen besagter Hydroperoxid-Zersetzungsreaktion in Gegenwart besagten so hergestellten Hydroperoxid-Zersetzungskatalysators in besagter Hydroperoxid-Zersetzungszone in flüssiger Phase unter Rühren unter Reaktionsbedingungen, die Temperatur im Bereich von 20° bis 40°C und autogenen Druck einschließen,

(c) kontinuierliches Entfernen eines Stroms besagten Hydroperoxid-Umwandlungsprodukts aus besagter Hydroperoxid-Umwandlungszone.

**26.** Verfahren nach Anspruch 17, bei dem besagtes t-Butylhydroperoxid kontinuierlich mit einer katalytisch wirksamen Menge eines Phthalocyanin-Katalysators in einer Reaktionszone unter Flüssigphasen-Reaktionsbedingungen, einschließlich einer Temperatur im Bereich von 20° bis 120°C und einem Druck von 0 bis 1.000 psig (6900 kPa g.p.) in Kontakt gebracht wird, dadurch gekennzeichnet, daß es folgendes umfaßt:

(a) Verwendung, als besagter Reduktionskatalysator, von 0,001 bis 5 Gew.-%, bezogen auf das Gewicht des Einsatzmaterials, eines Metall-Phthalocyanin-Katalysators, wobei das Metall besagten Metall-Phthalocyanin-Katalysators ein schweres Metall ist, das aus Gruppe IB, Gruppe VIIB und Gruppe VIIIB der Tabelle des Periodensystems ausgewählt ist, und

(b) Aktivieren der katalytischen Aktivität besagten Metall-Phthalocyanin-Katalysators mit von 0,2 bis 5 Gewichtsteilen, pro Teil besagten Metall-Phthalocyanin-Katalysators, Imidazol und

(c) kontinuierliches Entfernen eines Stroms besagten Umwandlungsprodukts aus besagter Reaktionszone.

**27.** Verfahren nach Anspruch 26, dadurch gekennzeichnet, daß der Strom kontinuierlich in eine Isobutan-Fraktion und eine t-Butylalkohol-Fraktion aufgetrennt wird, daß t-Butylalkohol aus besagter t-Butylalkoholfraktion entfernt wird und besagte Isobutan-Fraktion kontinuierlich zu besagter Reaktionszone rückgeführt wird.

**28.** Verfahren nach Anspruch 17, enthaltend eine Lösung von t-Butyl hydroperoxid in t-Butylalkohol mit 2 bis 90 Gew.-% t-Butylhydroperoxid, wobei nicht-umgesetztes Isobutan kontinuierlich aus besagter anfänglichen Reaktionsmischung abgetrennt wird, um ein Einsatzmaterial zur Verfügung zu stellen, das eine Lösung von besagtem t-Butylhydroperoxid in besagtem t-Butylalkohol zur Verfügung stellt, wobei besagtes Einsatzmaterial kontinuierlich einer Hydroperoxid-Zersetzungszone zugeführt wird und wobei eine katalytische Hydroperoxid-Zersetzungsreaktion kontinuierlich in besagter Zersetzungsreaktionszone durchgeführt wird, um besagtes t-Butylhydroperoxid zu Zersetzungsprodukten, hauptsächlich t-Butylalkohol, umzuwandeln, um ein Hydroperoxid-Umwandlungsprodukt zur Verfügung zu stellen, dadurch gekennzeichnet, daß es folgendes umfaßt:

(a) Zugabe von 0,001 bis 5 Gew.-%, bezogen auf das Gewicht des t-Butylhydroperoxids, Chloroferri-Phthalocyanin, 1 bis 1,5 Gewichtsteilen, pro Teil des Chloroferri-Phthalocyanins, 2,3-Dihydroxynaphthalin und 1 bis 20 Gewichtsteilen, pro Teil 2,3-dihydroxynaphthalin, eines $C_1$-$C_{18}$-Alkylthiols, zu besagtem Einsatzmaterial als besagter Hydroperoxid-Zersetzungskatalysator,

(b) Durchführen besagter Hydroperoxid-Zersetzungsreaktion in Gegenwart von besagtem so hergestellten Hydroperoxid-Zersetzungskatalysator in besagter Hydroperoxid-Zersetzungszone in flüssiger Phase unter Rühren unter Reaktionsbedingungen, die eine Temperatur im Bereich von 20° bis 125°C und autogenen Druck einschließen, und

(c) kontinuierliches Entfernen eines Stroms besagten Hydroperoxid-Umwandlungsprodukts aus besagter Hydroperoxid-Umwandlungszone.

**29.** Verfahren nach Anspruch 17, umfassend eine Lösung von t-Butylhydroperoxid in t-Butylalkohol, die von 5 bis 30 Gew.-% t-Butylhydroperoxid enthält, wobei nicht-umgesetztes Isobutan kontinuierlich von besagter anfänglichen Reaktionsmischung abgetrennt wird, um ein Einsatzmaterial zur Verfügung zu stellen, das eine Lösung von besagtem t-Butylhydroperoxid in besagten t-Butylalkohol umfaßt, besagtes Einsatzmaterial kontinuierlich einer Hydroperoxid-Zersetzungszone zugeführt wird und eine katalytische

Hydroperoxid-Zersetzungsreaktion kontinuierlich in besagter Zersetzungsreaktionszone durchgeführt wird, um besagtes t-Butylhydroperoxid zu Zersetzungsprodukten, hauptsächlich t-Butylalkohol, umzuwandeln, um ein Hydroperoxid-Umwandlungsprodukt zur Verfügung zu stellen, dadurch gekennzeichnet, daß es folgendes umfaßt:

(a) Zugabe von 0,001 bis 5 Gew.-%, bezogen auf das Gewicht des t-Butylhydroperoxids, Chloroferri-Phthalocyanin und 1 bis 5 Gewichtsteilen, bezogen auf das Gewicht des Chloroferri-Phthalocyanins, einer Alkalimetallbase einer organischen oder anorganischen Säure mit einem pH von mehr als 7,5, wenn 0,1 Mol in 1 Liter Wasser gelöst sind, zu besagtem Einsatzmaterial als besagten Hydroperoxid-Zersetzungskatalysator,

(b) Durchführen besagter Hydroperoxid-Zersetzungsreaktion in Gegenwart von besagten so hergestellten Hydroperoxid-Zersetzungskatalysator in besagter Hydroperoxid-Zersetzungszone in flüssiger Phase unter Rühren unter Reaktionsbedingungen, die eine Temperatur im Bereich von 20° bis 125°C und autogenen Druck einschließen,

(c) kontinuierliches Entfernen eines Stroms besagten Hydroperoxid-Umwandlungsprodukts aus besagter Hydroperoxid-Umwandlungszone.

**30.** Verfahren nach Anspruch 29, dadurch gekennzeichnet, daß besagtes Chloroferri-Phthalocyanin sich von Litium-, Natrium- oder r Kaliumacetat, -carbonat, -bicarbonat- oder -hydrogenphosphat ableitet.

**31.** Verfahren nach Anspruch 17, umfassend eine Lösung von t-Butylhydroperoxid und t-Butyl Alkohol, die von 2 bis 90 Gew.-% t-Butylhydroperoxid enthält, wobei nicht-umgesetztes Isobutan kontinuierlich von besagter anfänglicher Reaktionsmischung abgetrennt wird, um ein Einsatzmaterial zur Verfügung zu stellen, das eine Lösung von besagtem t-Butylhydroperoxid in besagtem t-Butylalkohol umfaßt, besagtes Einsatzmaterial kontinuierlich einer Hydroperoxid-Zersetzungszone zugeführt wird und eine katalytische Hydroperoxid-Zersetzungsreaktion kontinuierlich in besagter Zersetzungsreaktionszone durchgeführt wird, um besagtes t-Butylhydroperoxid zu Zersetzungsprodukten, hauptsächlich t-Butylalkohol, umzuwandeln, um ein Hydroperoxid-Umwandlungsprodukt zur Verfügung zu stellen, dadurch gekennzeichnet, daß es folgendes umfaßt:

(a) Zugabe von 0,001 bis 5 Gew.-%, bezogen auf das Gewicht des t-Butylhydroperoxids, Chloroferri-Phthalocyanin und etwa 1 bis 6 Gewichtsteilen, pro Teil Chloroferri-Phthalocyanin, eines Borats eines Metalls der Gruppe IA, Gruppe IIA oder Gruppe IIB der Tabelle des Periodensystems zu besagtem Einsatzmaterial als besagten Hydroperoxid-Zersetzungskatalysator,

(b) Durchführen besagter Hydroperoxid-Zersetzungsreaktion in Gegenwart besagten so hergestellten Hydroperoxid-Zersetzungskatalysators in besagter Hydroperoxid-Zersetzungszone in flüssiger Phase unter Rühren unter Reaktionsbedingungen, die eine Temperatur im Bereich von 20° bis 125°C und autogenen Druck einschließen,

(c) kontinuierliches Entfernen eines Stroms besagten Hydroperoxid-Umwandlungsprodukts aus besagter Hydroperoxid-Umwandlungszone.

**32.** Verfahren nach Anspruch 30, umfassend eine Lösung von t-Butylhydroperoxid in t-Butylalkohol, die von 20 bis 50 Gew.-% t-Butylhydroperoxid enthält, dadurch gekennzeichnet, daß es die Zugabe von (a) 1 bis 6 Gewichtsteilen, pro Teil des Chloroferri-Phthalocyanins, eines Litium-, Natrium-, Magnesium-, Zink- oder Calciumborats zu besagtem Einsatzmaterial als besagten Hydroperoxid-Zersetzungskatalysator umfaßt.

**33.** Verfahren nach Anspruch 26, dadurch gekennzeichnet, daß das Metall-Phthalocyanin Mangan-Phthalocyanin oder Chloroferri-Phthalocyanin ist.